# FASCICULE DE BREVET EUROPEEN

(11) **EP 0 888 306 B1**
(45) Date de publication et mention de la délivrance du brevet: **25.07.2001**
(21) Numéro de dépôt: 97914378.1
(22) Date de dépôt: 12.03.1997
(51) Int. Cl.: C07D 231/28, A61K 7/42, A61K 7/13

(54) **COMPOSITIONS COSMETIQUES A BASE DE PYRAZOLIN-4,5-DIONES, NOUVELLES PYRAZOLIN-4,5-DIONES, PROCEDES DE PREPARATION ET UTILISATIONS**
KOSMETISCHE ZUSAMMENSETZUNGEN AUF DER BASIS VON PYRAZOLIN-4,5-DIONEN,NEUE PYRAZOLIN-4,5-DIONEN,VERFAHREN ZU IHRER HERSTELLUNG UND IHRER VERWENDUNG
COSMETIC COMPOSITIONS CONTAINING PYRAZOLIN-4,5-DIONES, NOVEL PYRAZOLIN-4,5-DIONES, PREPARATION METHODS THEREFOR AND USES THEREOF

(30) Priorité: 22.03.1996 FR 9603625
(43) Date de publication de la demande: 07.01.1999
(73) Titulaire: L'OREAL, 75008 Paris (FR)
(72) Inventeur: MALLE, Gérard, F-77100 Meaux (FR); VIDAL, Laurent, F-75013 Paris (FR)
(74) Mandataire: Miszputen, Laurent
(86) Numéro de dépôt international: FR9700438
(87) Numéro de publication internationale: WO9735842

(56) Documents cités:
- FR-A- 2 466 492
- Tetrahedron (1994), Vol.50, No.3, 895-906 Chemical Abstracts (1977),Vol.86, No.19, Abstract 139927b

## Description

La présente invention concerne de nouvelles compositions cosmétiques, notamment pour colorer la peau et/ou les cheveux, comprenant, dans un support cosmétiquement acceptable, au moins une pyrazolin-4,5-dione.

L'invention se rapporte également à de nouvelles pyrazolin-4,5-diones. à leurs procédés de préparation et à leurs utilisations dans le domaine cosmétique, notamment pour colorer la peau et plus particulièrement en vue de donner à cette dernière un aspect de bronzage.

On sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence très proche de celui qui peut résulter d'une exposition prolongée au soleil (bronzage naturel) ou sous une lampe UV. Une telle utilisation présente en outre pour avantage d'éviter totalement les risques de réaction cutanée généralement attachés aux expositions prolongées précitées (érythèmes, brûlures, perte d'élasticité, apparition de rides, vieillissement prématuré de la peau, et autres).

Toutefois, l'utilisation de la DHA présente certains inconvénients.

En effet, bien que la couleur produite sur la peau par l'application d'une composition contenant de la DHA soit très proche de celle obtenue par un bronzage naturel, certains utilisateurs peuvent la juger encore trop jaune.

D'autres inconvénients apparaissent également au cours du stockage des compositions contenant de la DHA. Ainsi, cette dernière présente une fâcheuse tendance, plus ou moins prononcée selon la nature du milieu dans lequel elle est formulée, à se dégrader au cours du temps, cette dégradation se traduisant généralement à terme par un jaunissement non souhaitable des compositions qui la contiennent. Il arrive également qu'au cours du temps de telles compositions présentent une odeur nauséabonde. Enfin, le pH des compositions contenant de la DHA diminue au cours du temps, rendant celles-ci à terme incompatibles avec un emploi en application topique. Ces différents phénomènes font que l'activité de la DHA, et en particulier son aptitude à colorer la peau, est fortement diminuée au moment de l'application de ces compositions sur la peau.

Par ailleurs, l'intensité de la coloration obtenue sur la peau et surtout la rapidité avec laquelle cette coloration se développe sont souvent jugées très insuffisantes par les utilisateurs de produits autobronzants à base de DHA. En effet, la durée nécessaire à l'apparition sur la peau de l'intensité souhaitée est généralement de plusieurs heures.

En vue d'augmenter la rapidité de l'apparition de la couleur due à la DHA, on a cherché à associer cette dernière à d'autres substances. Ainsi, la demande EP-A-547864 propose de fournir la DHA en présence d'un aminoacide et d'une silicone, la DHA et l'aminoacide étant stockés avant application sur la peau dans des compartiments différents. On peut également citer la demande WO-A-9404130 qui décrit un dispositif pour fournir de la DHA en même temps qu'une amine primaire, ces deux composés étant stockés également dans des compartiments différents.

Toutefois, ces dispositifs présentent l'inconvénient d'être compliqués et de ne pas apporter de réelle amélioration quant à la durée d'attente nécessaire pour obtenir une coloration sur la peau satisfaisante. Enfin, ils ne résolvent pas non plus totalement les problèmes dus à la conservation des compositions contenant de la DHA.

En conclusion, il apparaît que la DHA à titre d'agent pour la coloration artificielle de la peau ne donne pas complètement satisfaction, et qu'il existe donc un besoin quant à pouvoir disposer d'autres agents qui, de préférence, ne présentent aucun des désavantages mentionnés ci-dessus.

A cet effet, il est maintenant proposé de nouvelles compositions contenant, dans un support cosmétiquement acceptable, au moins une pyrazolin-4,5-dione. Le temps nécessaire à la révélation de la couleur après application de ces compositions sur la peau est remarquablement court. Ces compositions présentent en outre une excellente stabilité et confèrent à la peau une couleur très proche de celle conférée par un bronzage naturel. Enfin, la tenue dans le temps de la coloration sur la peau est également remarquable.

Par ailleurs, ces compositions peuvent trouver une application intéressante dans la coloration capillaire.

L'invention a donc pour premier objet une nouvelle composition comprenant, dans un support cosmétiquement acceptable, au moins une pyrazolin-4,5 dione répondant à la formule (I) ci-dessous : 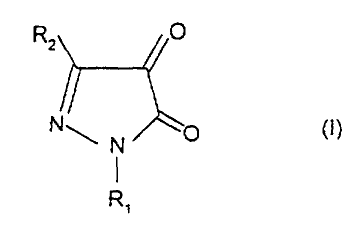 dans laquelle R₁ est choisi parmi :
- un atome d'hydrogène ; un radical alkyle, linéaire ou ramifié, en C₁-C₁₈ éventuellement substitué par un radical hydroxyle (OH), sulfonyle (SO₃H) ou carboxyle (COOH); un radical cyclohexyle ou cyclopentyle;
- un radical 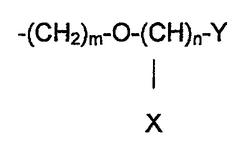 dans lequel m est égal à 1, 2 ou 3, n est égal à 1, 2 ou 3, X est un atome d'hydrogène ou un radical méthyle, et Y est un radical méthyle, hydroxy ou alcoxy en C₁-C₅, linéaire ou ramifié ;
- un radical -(CH₂)ₚ -OR' dans lequel R' représente un radical phényle ou naphtyle et p est égal à 1 ou 2 ;
- un radical -(CH₂)_{q}-R" dans lequel q est égal à 1,2 ou 3 et R" est choisi parmi:
   - un radical phényle substitué ou non par au maximum 2 radicaux choisis parmi les radicaux méthyle, trifluorométhyle, méthoxy ou sulfonyle ;
   - un radical naphtyle, un radical thiényle, un radical furyle, un radical pyridyle ou un radical pipéridyle ;
- un radical : 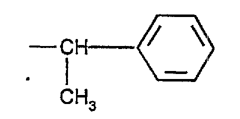
- un radical : 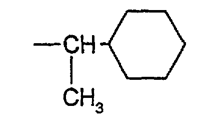
- un radical : 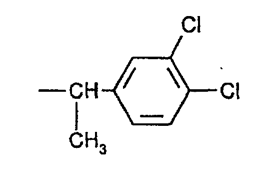
- un radical phényle ; un radical phényle substitué par un radical nitro ; un radical phényle substitué par un à trois radicaux choisis parmi : -COOH, -CH₂COOH, -Cl,
- Br, -F, -SO₃H, -CH₂OH, -OCF₃, -CF₃, -SO₂CH₃, -SO₂NH₂, -SO₂NHC₂H₅,-SO₂NHCH₂CH₂OH, -CON(CH₃)₂, -CON(C₂H₅)₂, -CH₂N(CH₃)₂, -CH₂N(C₂H₅)₂, -NHCOCH₃, -NHCOC₂H₅, un radical alkyle en C₁-C₈ ou -ZR₃ dans lequel Z est un atome d'oxygène ou de soufre et R₃ est un atome d'hydrogène ou un radical alkyle linéaire ou ramifié en C₁-C₁₈;
- un radical naphtyle éventuellement substitué par un radical -SO₃H;
- un radical benzyle substitué par un radical -COOH;
- un radical pyridyle, un radical pyrimidinyle, un radical pyrazinyle, un radical triazinyle, un radical benzotriazolyle, un radical benzimidazolyle, un radical thiényle, un radical imidazolyle, un radical thiazolyle, un radical triazolyle-1,2,4, un radical indazolyle, un radical indolyle, un radical quinolyle ou un radical isoquinolyle ;
et R₂ est choisi parmi :
- un atome d'hydrogène ; un radical alkyle en C₁-C₆, linéaire ou ramifié ; un radical hydroxyalkyle en C₁-C₄;
- un radical phényle ; un radical phényle substitué par un atome d'halogène, un radical nitro ou un radical trifluorométhyle ; un radical phényle substitué par au maximum trois radicaux choisis parmi les radicaux alkyle en C₁-C₄, alcoxy en C₁-C₄, dialkylamino en C₁-C₄ ou alkylthio en C₁-C₂;
- un radical benzyle ; un radical benzyle substitué par un atome d'halogène, un radical alkyle en C₁-C₄, un radical alcoxy en C₁-C₄, un radical trifluorométhyle ou un radical dialkylamino en C₁-C₄;
- un radical -(CH₂)ᵣ-R₄ dans lequel r est égal à 1,2 ou 3 et R₄ est choisi parmi :
   - un radical -SO₃H, un radical akylthio en C₁-C₂ ou un radical benzylthio ;
      - un radical méthoxycarbonyle ou éthoxycarbonyle ;
   - un radical alcoxy en C₁-C₄ ou un radical phénoxy éventuellement substitué par un ou plusieurs atomes d'halogène;
- un radical alcoxy en C₁-C₄; un radical trifluorométhyle ; acétamido ; dialkylamino en C1-C4 ; carboxy ; méthoxycarbonyle ; éthoxycarbonyle ;
- un radical thiényle, un radical furyle, un radical pyridyle ou un radical pyrazolyle.

Dans une forme préférée de réalisation de l'invention, ces compositions sont destinées au bronzage artificiel de la peau.

Ces nouvelles compositions présentent l'avantage de conférer à la peau un bronzage très proche du bronzage naturel et ce, en un temps extrêmement court. En effet, quelques minutes suffisent pour obtenir un teint bronzé.

La présente invention a encore pour objet un procédé de traitement cosmétique de la peau destiné à la bronzer et/ou la brunir artificiellement, consistant à appliquer sur celle-ci une quantité efficace d'une composition cosmétique conforme à l'invention ou d'une pyrazolin-4,5-dione telle que mentionnée ci-dessus.

La présente invention a aussi pour objet l'utilisation d'une pyrazolin-4,5-dione telle que mentionnée ci-dessus dans, ou pour la fabrication de, compositions destinées au bronzage et/ou au brunissage artificiels de la peau.

Dans une autre forme de réalisation de l'invention, ces compositions cosmétiques sont destinées à colorer les cheveux.

La présente invention a ainsi également pour objet un procédé de traitement cosmétique des cheveux destiné à les colorer, consistant à appliquer sur les cheveux une quantité efficace d'une composition conforme à l'invention ou d'une pyrazolin-4,5-dione telle que mentionnée ci-dessus.

La présente invention a encore pour objet l'utilisation d'une pyrazolin-4,5-dione telle que mentionnée ci-dessus dans, ou pour la fabrication de, compositions destinées à colorer les cheveux.

D'autres caractéristiques, aspects et avantages de la présente invention apparaîtront à la lecture de la description détaillée qui va suivre.

Parmi les pyrazolin-4,5-diones définies ci-dessus, on préfère plus particulièrement celles pour lesquelles R₁ est choisi parmi :
- un atome d'hydrogène ; un radical alkyle, linéaire ou ramifié, en C1-C8,
- un radical -(CH2)₂-OR' dans lequel R' représente un radical phényle ou naphtyle ;
- un radical -(CH2)q-R" dans lequel q est égal à 1 ou 2 et R" représente un radical phényle éventuellement substitué par un radical trifluorométhyle ;
- un radical phényle éventuellement substitué par un radical nitro, un radical -Cl, un radical alkyle en C1-C4 ou un radical alcoxy en C1-C4,
et celles pour lesquelles R₂ est choisi parmi :
- un atome d'hydrogène ; un radical alkyle en C1-C4, linéaire ou ramifié ; un radical hydroxyalkyle en C1-C4 ; un radical méthoxyméthyle ;
- un radical phényle éventuellement substitué par un atome d'halogène, un radical nitro, un radical alkyle en C1-C4 ou un radical alcoxy en C1-C4 ;
- un radical alcoxy en C1-C4 ; un radical trifluorométhyle ; acétamido ; dialkylamino en C1-C4 ; carboxy ; méthoxycarbonyle ; éthoxycarbonyle ;
- un radical thiényle, un radical furyle, un radical pyridyle ou un radical pyrazolyle,
les conditions ci-dessus pour le choix de R₁ et R₂ étant, de préférence, cumulatives.

Ainsi, parmi les pyrazolin-4,5-diones utilisables dans les compositions conformes à l'invention, on peut notamment citer :
- la 3-méthyl 1-phényl pyrazolin-4,5-dione ; la 1-phényl pyrazolin-4,5-dione ; la 3-tert-butyl 1-phényl pyrazolin-4,5-dione ; la 1,3-diphényl pyrazolin-4,5-dione ; la 1-phényl 3-(4'-méthylphényl) pyrazolin-4,5-dione; la 1-phényl 3-(3'-méthoxyméthyl) pyrazolin-4,5-dione, la 1-phényl 3-(4'-méthoxyphényl) pyrazolin -4,5-dione ; la 1-phényl 3-(4'-nitrophényl) pyrazolin-4,5-dione; la 3-mélhoxy 1-phényl pyrazolin-4,5-dione ; la 3-éthoxy 1-phényl pyrazolin-4,5-dione; la 3-acétamido 1-phényl pyrazolin-4,5-dione ; la 3-diméthylamino 1-phényl pyrazolin-4,5-dione; la 3-diéthylamino 1-phényl pyrazolin-4,5-dione ; la 3-carboxy 1-phényl pyrazolin-4,5-dione ; la 3-méthoxycarbonyl 1-phényl pyrazolin-4,5-dione; la 3-éthoxycarbonyl 1-phényl pyrazolin-4,5-dione ;
- la 1-[(3'-trifluorométhyl)benzyl)] 3-méthyl pyrazolin-4,5-dione ; la 1-[(1'-phényl)éthyl)] 3-méthyl pyrazolin-4,5-dione ; la 3-méthyl pyrazolin-4,5-dione; la 1,3-diméthyl pyrazolin -4,5-dione ; la 1-(2'-phénoxy)éthyl 3-méthyl pyrazolin-4,5-dione ; la 1-(2'-naphtyloxy)éthyl 3-propyl pyrazolin-4,5-dione; la 1-(2'-naphtyloxy) éthyl 3-hydroxyméthyl pyrazolin-4,5-dione; la 3-tert-butyl 1-(2'-phénoxy) éthyl pyrazolin-4,5-dione ; la 3-méthoxyméthyl 1-(2'-naphtyloxy) éthyl pyrazolin-4,5-dione ; la 3-méthyl 1-(4'-nitrophényl) pyrazolin-4,5-dione ; la 3-méthoxy pyrazolin-4,5-dione ; la 3-éthoxy pyrazolin-4,5-dione ;
- la 1-méthyl pyrazolin-4,5-dione; la 1-méthyl 3-phényl pyrazolin-4,5-dione ; la 1-méthyl 3-(4'-chlorophényl) pyrazolin-4,5-dione ; la 1-méthyl 3-(3'-méthoxyphényl) pyrazolin-4,5-dione ; la 1-méthyl 3-(4'-méthoxyphényl) pyrazolin-4,5-dione ; la 1-méthyl 3-(3'-nitrophényl) pyrazolin-4,5-dione ; la 1-méthyl 3-(4'-méthylphényl) pyrazolin-4,5-dione ; la 1-méthyl 3-(2'-furyl) - pyrazolin-4,5-dione ; la 1-méthyl 3-(2'-thienyl) - pyrazolin-4,5-dione; la 1-méthyl 3-(5'-pyrazolyl) pyrazolin-4,5-dione ; la 1-méthyl 3-(4'-pyridyl) pyrazolin-4,5-dione ; la 1-méthyl 3-méthoxy pyrazolin-4,5-dione ; la 3-éthoxy 1-méthyl pyrazolin-4,5-dione ; la 3-diméthylamino 1-méthyl pyrazolin-4,5-dione; la 3-diéthylamino 1-méthyl pyrazolin-4,5-dione ; la 3-acétamido 1-méthyl pyrazolin-4,5-dione ; la 3-carboxy 1-méthyl pyrazolin-4,5-dione ; la 3-méthoxycarbonyle 1-méthyl pyrazolin-4,5-dione ; la 3-éthoxycarbonyle 1-méthyl pyrazolin-4,5-dione ;
- la 1-éthyl pyrazolin-4,5-dione ; la 1-éthyl 3 -méthyl pyrazolin-4,5-dione; la 1-éthyl 3 -phényl pyrazolin-4,5-dione ; la 1-éthyl 3-(4'-chlorophényl) pyrazolin-4,5-dione ; la 1-éthyl 3-(3'méthoxyphényl) pyrazolin-4,5-dione ; la 1-éthyl 3-(4'-méthoxyphényl) pyrazolin-4,5-dione ; la 1-éthyl 3-(3'-nitrophényl) pyrazolin-4,5-dione ; la 1-éthyl 3-(4'-méthylphényl) pyrazolin-4,5-dione ; la 1-éthyl 3-(2'-furyl) pyrazolin-4,5-dione; la 1-éthyl 3-(2'-thiényl) pyrazolin-4,5-dione; la 1-éthyl 3-(5'-pyrazolyl) pyrazolin-4,5-dione ; la 1-éthyl 3-méthoxy pyrazolin-4,5-dione; la 1-éthyl 3-éthoxy pyrazolin-4,5-dione; la 1-éthyl 3-diméthylamino pyrazolin-4,5-dione ; la 1-éthyl 3-diéthylamino pyrazolin-4,5-dione ; la 1-éthyl 3-acétamido pyrazolin-4,5-dione ; la 1-éthyl 3-carboxy pyrazolin-4,5-dione; la 1-éthyl 3-méthoxycarbonyl pyrazolin-4,5-dione; la 1-éthyl 3-éthoxycarbonyl pyrazolin-4,5-dione ;
- la 1-isopropyl pyrazolin-4,5-dione; la 1-isopropyl 3-méthyl pyrazolin-4,5-dione ; la 1-isopropyl 3 -phényl pyrazolin-4,5-dione; la 1- isopropyl 3-(4'-chlorophényl) pyrazolin-4,5-dione ; la 1-isopropyl 3-(3'-méthoxyphényl) pyrazolin-4,5-dione ; la 1- isopropyl 3-(4'-méthoxyphényl) pyrazolin-4,5-dione; la 1- isopropyl 3-(3'-nitrophényl) pyrazolin-4,5-dione ; la 1- isopropyl 3-(4'-méthylphényl) pyrazolin-4,5-dione ; la 1- isopropyl 3-(2'-furyl) pyrazolin-4,5-dione ; la 1- isopropyl 3-(2'-thiényl) pyrazolin-4,5-dione; la 1-isopropyl 3-(5'-pyrazolyl) pyrazolin-4,5-dione ; la 1-isopropyl 3-méthoxy pyrazolin-4,5-dione ; la 1- isopropyl 3-éthoxy pyrazolin-4,5-dione ; la 1- isopropyl 3-diméthylamino pyrazolin-4,5-dione ; la 1- isopropyl 3-diéthylamino pyrazolin-4,5-dione ; la 1- isopropyl 3-acétamido pyrazolin-4,5-dione; la 1- isopropyl 3-carboxy pyrazolin-4,5-dione ; la 1- isopropyl 3-méthoxycarbonyl pyrazolin-4,5-dione; la 1- isopropyl 3-éthoxycarbonyl pyrazolin-4,5-dione ;
- la 1-tert-butyl pyrazolin-4,5-dione ; la 1-tert-butyl 3-méthyl pyrazolin-4,5-dione ; la 1-tert-butyl 3- phényl pyrazolin-4,5-dione ; la 1-tert-butyl 3-(4'-chlorophényl) pyrazolin-4,5-dione; la 1-tert-butyl 3-(3'-méthoxyphényl) pyrazolin-4,5-dione ; la 1-tert-butyl 3-(4'-méthoxyphényl) pyrazolin-4,5-dione ; la 1-tert-butyl 3-(3'-nitrophényl) pyrazolin-4,5-dione; la 1-tert-butyl 3-(4'-méthylphényl) pyrazolin-4,5-dione ; la 1-tert-butyl 3-(2'-furyl) pyrazolin-4,5-dione ; la 1-tert-butyl 3-(2'-thiényl) pyrazolin-4,5-dione ; la 1-tert-butyl 3-(5'-pyrazolyl) pyrazolin-4,5-dione; la 1- tert-butyl 3-méthoxy pyrazolin-4,5-dione ; la 1- tert-butyl 3-éthoxy pyrazolin-4,5-dione ; la 1- tert-butyl 3-diméthylamino pyrazolin-4,5-dione ; la 1- tert-butyl 3-diéthylamino pyrazolin-4,5-dione ; la 1- tert-butyl 3-acétamido pyrazolin-4,5-dione ; la 1- tert-butyl 3-carboxy pyrazolin-4,5-dione; la 1- tert-butyl 3-méthoxycarbonyl pyrazolin-4,5-dione ; la 1- tert-butyl 3-éthoxycarbonyl pyrazolin-4,5-dione ;
- la 1-octyl pyrazolin-4,5-dione ; la 1-octyl 3-méthyl pyrazolin-4,5-dione ; la 1-octyl 3-phényl pyrazolin-4,5-dione; la 1-octyl 3-(4'-chlorophényl) pyrazolin-4,5-dione ; la 1-octyl 3-(3'-méthoxyphényl) pyrazolin-4,5-dione ; la 1-octyl 3-(4'-méthoxyphényl) pyrazolin-4,5-dione ; la 1-octyl 3-(3'-nitrophényl) pyrazolin-4,5-dione ;
- la 1-(4'-méthylphényl) pyrazolin-4,5-dione; la 1-(4'-méthylphényl) 3-méthyl pyrazolin-4,5-dione ; la 1-(4'-méthylphényl) 3 phényl pyrazolin-4,5-dione; la 1-(4'-méthylphényl) 3-(4'-chlorophényl) pyrazolin-4,5-dione ; la 1-(4'-méthylphényl) 3-(3'-méthoxyphényl) pyrazolin-4,5-dione; la 1-(4'-méthylphényl) 3-(4'-méthoxyphényl) pyrazolin-4,5-dione ; la 1-(4'-méthylphényl) 3-(3'-nitrophényl) pyrazolin-4,5-dione ; la 1-(4'-méthylphényl) 3-(4'-méthylphényl) pyrazolin-4,5-dione ; la 1-(4'-méthylphényl) 3-(2'-furyl) pyrazolin-4,5-dione; 1-(4'-méthylphényl) 3-(2'-thiényl) pyrazolin-4,5-dione ; la 1-(4'-méthylphényl) 3-(5'-pyrazolyl) pyrazolin-4,5-dione ; la 1-(4'-méthylphényl) 3-méthoxy pyrazolin-4,5-dione ; la 1-(4'-méthylphényl) 3-éthoxy pyrazolin-4,5-dione ; la 1-(4'-méthylphényl) 3-diméthylamino pyrazolin-4,5-dione ; la 1-(4'-méthylphényl) 3-diéthylamino pyrazolin-4,5-dione ; la 1-(4'-méthylphényl) 3-acétamido pyrazolin-4,5-dione ; la 1-(4'-méthylphényl) 3-carboxy pyrazolin-4,5-dione ; la 1-(4'-méthylphényl) 3-méthoxycarbonyl pyrazolin-4,5-dione ; la 1-(4'-méthylphényl) 3-éthoxycarbonyl pyrazolin-4,5-dione ;
- la 1-benzyl pyrazolin-4,5-dione; la 1-benzyl 3-méthyl pyrazolin-4,5-dione ; la 1-benzyl 3-phényl pyrazolin-4,5-dione ; la 1-benzyl 3-(4'méthylphényl) pyrazolin-4,5-dione ; la 1-benzyl 3-(3'-méthoxyphényl) pyrazolin-4,5-dione; la 1-benzyl 3-(4'-méthoxyphényl) pyrazolin-4,5-dione ; la 1-benzyl 3-(3'-nitrophényl) pyrazolin-4,5-dione ;
- la 1-(4'-méthoxyphényl) pyrazolin-4,5-dione ; la 1-(4'-méthoxyphényl) 3-méthyl pyrazolin-4,5-dione ; la 1-(4'-méthoxyphényl) 3-(3'-méthoxyphényl) pyrazolin-4,5-dione ; la 1-(4'-méthoxyphényl) 3-phényl pyrazolin-4,5-dione ; la 1-(4'-méthoxyphényl) 3-(4'-chlorophényl) pyrazolin-4,5-dione ; la 1-(4'-méthoxyphényl) 3-(4'-méthoxyphényl) pyrazolin-4,5-dione; la 1-(4'-méthoxyphényl) 3-(3'-nitrophényl) pyrazolin-4,5-dione; la 1-(4'-méthoxyphényl) 3-méthoxy pyrazolin-4,5-dione ; la 1-(4'-méthoxyphényl) 3-éthoxy pyrazolin-4,5-dione ; la 1-(4'-méthoxyphényl) 3-diméthylamino pyrazolin-4,5-dione ; la 1-(4'-méthoxyphényl) 3-diéthylamino pyrazolin-4,5-dione ; la 1-(4'-méthoxyphényl) 3-acétamido pyrazolin-4,5-dione ; la 1-(4'-méthoxyphényl) 3-carboxy pyrazolin-4,5-dione ; la 1-(4'-méthoxyphényl) 3-méthoxycarbonyl pyrazolin-4,5-dione ; la 1-(4'-méthoxyphényl) 3-éthoxycarbonyl pyrazolin-4,5-dione ;
- la 1-(4'chlorophényl) pyrazolin-4,5-dione ; la 1-(4'-chlorophényl) 3-méthyl pyrazolin-4,5-dione ; la 1-(4'-chlorophényl) 3-phényl pyrazolin-4,5-dione ; la 1-(4'-chlorophényl) 3-(4'-méthylphényl) pyrazolin-4,5-dione; la 1-(4'-chlorophényl) 3-(3'-méthoxyphényl) pyrazolin-4,5-dione ; la 1-(4'-chlorophényl) 3-(4'-méthoxyphényl) pyrazolin-4,5-dione ; la 1-(4'-chlorophényl) 3-(3'-nitrophényl) pyrazolin-4,5-dione ; la 1-(4'-chlorophényl) 3-méthoxy pyrazolin-4,5-dione ; la 1-(4'-chlorophényl) 3-éthoxy pyrazolin-4,5-dione ; la 1-(4'-chlorophényl) 3-diméthylamino pyrazolin-4,5-dione; la 1-(4'-chlorophényl) 3-diéthylamino pyrazolin-4,5-dione ; la 1-(4'-chlorophényl) 3-acétamido pyrazolin-4,5-dione ; la 1-(4'-chlorophényl) 3-carboxy pyrazolin-4,5-dione ; la 1-(4'-chlorophényl) 3-méthoxycarbonyl pyrazolin-4,5-dione ; la 1-(4'-chlorophényl) 3-éthoxycarbonyl pyrazolin-4,5-dione ;
- la 1-(4'-nitrophényl) pyrazolin-4,5-dione; la 1-(4'-nitrophényl) 3-méthyl pyrazolin-4,5-dione ; la 1-(4'-nitrophényl) 3-phényl pyrazolin-4,5-dione ; la 1-(4'-nitrophényl) 3-(4'-méthylphényl) pyrazolin-4,5-dione ; la 1-(4'-nitrophényl) 3-(3'-méthoxyphényl) pyrazolin-4,5-dione ; la 1-(4'-nitrophényl) 3-(4'-méthoxyphényl) pyrazolin-4,5-dione ; la 1-(4'-nitrophényl) 3-(3'-nitrophényl) pyrazolin-4,5-dione ;
- la 1-phényl 3-trifluorométhyl pyrazolin-4,5-dione; la 1-méthyl 3-trifluorométhyl pyrazolin-4,5-dione ; la 1-isopropyl 3-trifluorométhyl pyrazolin-4,5-dione ; la 1-éthyl 3-trifiuorométhyl pyrazolin-4,5-dione ; la 3-trifluorométhyl pyrazolin-4,5-dione.

Parmi les pyrazolin-4,5-diones utilisables dans le cadre de la présente invention, on préfère tout particulièrement celles pour lesquelles, cumulativement, R₁ est choisi parmi :
- l'hydrogène et les radicaux méthyle, éthyle, isopropyle, tertiobutyle ou phényle;
et R₂ est choisi parmi :
- l'hydrogène ou les radicaux méthyle, phényle, méthoxyphényle, méthoxy, éthoxy, carboxy, méthoxycarbonyle, éthoxycarbonyle, acétamido, diméthylamino, diéthylamino, trifluorométhyle, furyle, pyridyle.

Ainsi, dans les compositions selon l'invention, on préfère plus particulièrement utiliser la 3-méthyl 1-phényl pyrazolin-4,5-dione ; la 3-méthyl pyrazolin-4,5-dione ; la 1,3-diméthyl pyrazolin-4,5-dione ; la 1-éthyl 3-méthyl pyrazolin-4,5-dione ; la 1-isopropyl 3-méthyl pyrazolin-4,5-dione ; la 1-tertbutyl 3-méthyl pyrazolin-4,5-dione ; la 1-méthyl 3-phényl pyrazolin-4,5-dione ; la 1-méthyl 3-(3'-méthoxyphényl) pyrazolin-4,5-dione; la 3(2'-furyl) 1-méthyl pyrazolin-4,5-dione; la 1-méthyl 3-(4'-pyridyl) pyrazolin-4,5-dione; la 1-méthyl 3-méthoxy pyrazolin-4,5-dione; la 3-éthoxy 1-méthyl pyrazolin-4,5-dione ; la 3-diméthylamino 1-méthyl pyrazolin-4,5-dione ; la 3-diéthylamino 1-méthyl pyrazolin-4,5-dione; la 3-acétamido 1-méthyl pyrazolin-4,5-dione; la 1-phényl pyrazolin-4,5-dione ; la 1-méthyl pyrazolin-4,5-dione ; la 1-éthyl pyrazolin-4,5-dione; la 1-isopropyl pyrazolin-4,5-dione; la 1-tertbutyl pyrazolin-4,5-dione ; la 3-méthoxy 1-phényl pyrazolin-4,5-dione; la 3-éthoxy 1-phényl pyrazolin-4,5-dione ; la 3-acétamido 1-phényl pyrazolin-4,5-dione ; la 3-diméthylamino 1-phényl pyrazolin-4,5-dione ; la 3-diéthylamino 1-phényl pyrazolin-4,5-dione ; la 1-phényl 3-trifluorométhyl pyrazolin-4,5-dione; la 1-méthyl 3-trifluorométhyl pyrazolin-4,5-dione ; la 1-isopropyl 3-trifluorométhyl pyrazolin-4,5-dione ; la 1-éthyl 3-trifluorométhyl pyrazolin-4,5-dione; la 3-trifluorométhyl pyrazolin-4,5-dione ; la 3-carboxy 1-phényl pyrazolin-4,5-dione ; la 3-méthoxycarbonyl 1-phényl pyrazolin-4,5-dione ; la 3-éthoxycarbonyl 1-phényl pyrazolin-4,5-dione; la 3-méthoxy pyrazolin-4,5-dione ; la 3-éthoxy pyrazolin-4,5-dione ; la 3-carboxy 1-méthyl pyrazolin-4,5-dione ; la 3-méthoxycarbonyle 1-méthyl pyrazolin-4,5-dione ; la 3-éthoxycarbonyle 1-méthyl pyrazolin-4,5-dione.

La ou les pyrazolin-4,5-diones sont utilisées en une quantité efficace pour conférer une coloration soit sur la peau soit sur les cheveux, selon les cas. Ainsi, elles sont généralement présentes dans les compositions selon l'invention à une concentration comprise entre 0,05 et 10 % en poids, de préférence entre 0,05 et 5 % en poids, par rapport au poids total de la composition.

Lorsque les compositions selon l'invention sont destinées au bronzage artificiel de la peau, le pH peut varier entre 3 et 10. Il est de préférence compris entre 3 et 7.

Lorsque les compositions selon l'invention sont destinées à la coloration des cheveux, le pH peut varier entre 2 et 8. Il est de préférence compris entre 2 et 6.

Le véhicule (ou support cosmétiquement acceptable) des compositions selon l'invention est de préférence l'eau, seule ou avec un ou plusieurs solvants organiques ou corps gras.

Les solvants utilisables sont de préférence choisis parmi les alcools tels que l'alcool éthylique ou l'alcool cétylique, ou encore parmi le propylène glycol, l'éthylène glycol monoéthyléther et l'éthylène glycol monobutyléther.

Comme corps gras, on peut citer les huiles et les cires d'origine minérale, animale ou végétale.

Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis) ; synthétiques comme le perhydrosqualène, les alcools, les acides ou les esters gras (palmitate d'octyle, lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer l'huile de jojoba, la paraffine, la cire de carnauba, la cire d'abeille, l'huile de ricin hydrogénée.

Les autres constituants pouvant rentrer dans la formulation des compositions visées par l'invention, en particulier les épaississants, les émulsionnants, les gélifiants sont ceux qui sont classiquement utilisés dans le domaine cosmétique et/ou dermatologique.

Comme émulsionnants, on peut utiliser les esters d'acides gras et de polyéthylène glycol (PEG), les esters d'acides gras et de glycérol (stéarate de glycéryle) ou les esters d'acides gras et de sucre (stéarate de sorbitane), ainsi que leurs dérivés polyoxyéthylénés ou polyoxypropylénés, les cyclométhicones et diméthicones copolyols, les tensioactifs anioniques (alkylphosphate de K ou de Na), les alcools gras polyalcoxylés.

De préférence, on utilise les alcools gras polyalcoxylés tels que les alcools butyliques oxypropylénés, les alcools capryliques oxyéthylénés, les alcools cétyliques oxyéthylénés.

Comme épaississants, on peut utiliser les acides polyacryliques réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Comme gélifiants, on peut citer les argiles modifiées (bentones), les sels métalliques d'acides gras (stéarate d'aluminium), les copolymères éthylène/acrylate, les silices, les polyéthylènes, les silicates de calcium ou encore l'éthylcellulose.

Les compositions selon l'invention peuvent en outre contenir tout autre constituant cosmétiquement ou dermatologiquement acceptable habituellement utilisé dans ce genre de compositions, tels que les adoucissants, les antioxydants, les opacifiants, les stabilisants, les émollients, les agents répulsifs contre les insectes, les filtres solaires organiques actifs dans l'UV-A et/ou l'UV-B, les pigments et les nanopigments minéraux photoprotecteurs, les agents hydratants, les vitamines, les parfums, les conservateurs, les charges, les séquestrants, les colorants, et en particulier les composés connus pour leur action auto-bronzante comme par exemple la dihydroxyacétone, le méthylglycoxal, l'aldéhyde glycérique, l'érythrulose, l'alloxane, le dialdéhyde 2,3-dihydroxysuccinique, le dialdéhyde 2-amino-3-hydroxysuccinique, le dialdéhyde 2-benzylamino-3-hydroxysuccinique.

Bien entendu, l'homme de l'art veillera à choisir ce ou ces éventuels composés complémentaires et/ou leurs quantités de manière telle que les propriétés avantageuses attachées intrinsèquement à la composition conforme à l'invention ne soient pas, ou substantiellement pas, altérées par la ou les adjonctions envisagées.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

De préférence, cette composition se présente sous la forme d'une émulsion huile-dans-eau.

Les compositions concernées par l'invention peuvent être préparées selon les techniques bien connues de l'homme de l'art, en particulier celles destinées à la préparation d'émulsions de type huile-dans-eau ou eau-dans-huile.

Certaines pyrazolin-4,5-diones de formule (I) sont connues. Elles sont notamment divulguées dans Tetrahedron Vol. 50, No. 3, pp. 895-906, 1994 et Cheminal Abstracts Vol. 86, No. 19, Abrégé 139927b, 1977.

La présente invention a également pour objet de nouvelles pyrazolin-4,5-diones répondant à la formule générale (I') suivante : 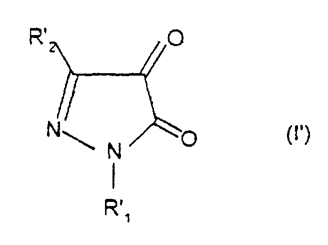 dans laquelle R'₁ et R'₂ ont les mêmes significations que celles indiquées précédemment pour R₁ et R₂ dans la formule (I) avec néanmoins les réserves suivantes :
- (i) lorsque R'₁ représente un radical phényle, alors R'₂ est différent d'un atome d'hydrogène, d'un radical méthyle, d'un radical tertiobutyle, d'un radical phényle, d'un radical p-méthylphényle, d'un radical p-nitrophényle ou d'un radical p-méthoxyphényle,
- (ii) lorsque R'₂ représente un radical méthyle, R'₁ est différent d'un radical p-nitrophényle, d'un radical 2-phénoxyéthyle, d'un radical 2-naphtyloxyéthyle, d'un radical méthyle, d'un radical m-trifluorométhylbenzyle, d'un radical 1-phényléthyle, d'un radical p-méthylphényle, d'un radical 1-cyclohexyléthyle ou d'un radical 1-(3',4'-dichlorophényl) éthyle,
- (iii) lorsque R'₁ représente un radical 2-naphtyloxyéthyle, R'₂ est différent d'un radical n-propyle ou d'un radical hydroxyméthyle,
- (iv) lorsque R'₁ représente un radical phénoxy-2 éthyle, R'₂ est différent d'un radical tertiobutyle,
- (v) lorsque R'₂ représente un radical méthoxyméthyle, R'₁ est différent d'un radical 2-naphtyloxyéthyle ou d'un radical 1-(3',4'-dichlorophényl) éthyle,
- (vi) lorsque R'₁ représente un atome d'hydrogène, R'₂ est différent d'un radical méthyle,
- (vii) lorsque R'₂ représente un radical phényle, R'₁ est différent d'un radical méthyle, d'un radical p-méthoxyphényle ou d'un radical p-nitrophényle.

Parmi les pyrazolin-4,5-diones définies ci-dessus, on peut plus particulièrement citer celles pour lesquelles R'₁ est choisi parmi :
- un atome d'hydrogène ; un radical alkyle, linéaire ou ramifié, en C1-C8,
- un radical -(CH2)₂-OR' dans lequel R' représente un radical phényle ou naphtyle ;
- un radical -(CH2)q-R" dans lequel q est égal à 1 ou 2 et R" représente un radical phényle éventuellement substitué par un radical trifluorométhyle ;
- un radical phényle éventuellement substitué par un radical nitro, un radical -Cl, un radical alkyle en C1-C4 ou un radical alcoxy en C1-C4,
et celles pour lesquelles R'₂ est choisi parmi :
- un atome d'hydrogène ; un radical alkyle en C1-C4, linéaire ou ramifié ; un radical hydroxyalkyle en C1-C4 ; un radical méthoxyméthyle ;
- un radical phényle éventuellement substitué par un atome d'halogène, un radical nitro, un radical alkyle en C1-C4 ou un radical alcoxy en C1-C4 ;
- un radical alcoxy en C1-C4 ; un radical trifluorométhyle ; acétamido ; dialkylamino en C1-C4 ; carboxy ; méthoxycarbonyle ; éthoxycarbonyle ;
- un radical thiényle, un radical furyle, un radical pyridyle ou un radical pyrazolyle,
avec les réserves (i) à (vi) définies ci-dessus, les conditions ci-dessus pour le choix de R'₁ et R'₂ étant, de préférence, cumulatives.

Parmi les nouveaux composés de formule (I'), on peut ainsi tout particulièrement citer ceux pour lesquels R₁' est choisi parmi :
- l'hydrogène ou les radicaux méthyle, éthyle, isopropyle, tertiobutyle ou phényle;
et R₂' est choisi parmi :
- l'hydrogène ou les radicaux méthyle, phényle, méthoxyphényle, méthoxy, éthoxy, carboxy, méthoxycarbonyle, éthoxycarbonyle, acétamido, diméthylamino, diéthylamino, trifluorométhyle, furyle, pyridyle,
avec les réserves (i) à (vi) définies ci-dessus.

Ainsi, on peut notamment citer :
- la 1-éthyl 3-méthyl pyrazolin-4,5-dione ; la 1-isopropyl 3-méthyl pyrazolin-4,5-dione ; la 1-tertbutyl 3-méthyl pyrazolin-4,5-dione; la 1-méthyl 3-(3'-méthoxyphényl) pyrazolin-4,5-dione; la 3(2'-furyl) 1-méthyl pyrazolin-4,5-dione ; la 1-méthyl 3-(4'-pyridyl) pyrazolin-4,5-dione ; la 1-méthyl 3-méthoxy pyrazolin-4,5-dione ; la 3-éthoxy 1-méthyl pyrazolin-4,5-dione ; la 3-diméthylamino 1-méthyl pyrazolin-4,5-dione; la 3-diéthylamino 1-méthyl pyrazolin-4,5-dione ; la 3-acétamido 1-méthyl pyrazolin-4,5-dione ; la 1-méthyl pyrazolin-4,5-dione ; la 1-éthyl pyrazolin-4,5-dione; la 1-isopropyl pyrazolin-4,5-dione; la 1-tertbutyl pyrazolin-4,5-dione ; la 3-méthoxy 1-phényl pyrazolin-4,5-dione ; la 3-éthoxy 1-phényl pyrazolin-4,5-dione; la 3-acétamido 1-phényl pyrazolin-4,5-dione ; la 3-diméthylamino 1-phényl pyrazolin-4,5-dione ; la 3-diéthylamino 1-phényl pyrazolin-4,5-dione ; la 1-phényl 3-trifluorométhl pyrazolin-4,5-dione ; la 1-méthyl 3-trifluorométhyl pyrazolin-4,5-dione : la 1-isopropyl 3-trifluorométhyl pyrazolin-4,5-dione ; la 1-éthyl 3-trifluorométhyl pyrazolin-4,5-dione ; la 3-trifluorométhyl pyrazolin-4,5-dione ; la 3-carboxy 1-phényl pyrazolin-4,5-dione ; la 3-méthoxycarbonyl 1-phényl pyrazolin-4,5-dione ; la 3-éthoxycarbonyl 1-phényl pyrazolin-4,5-dione; la 3-méthoxy pyrazolin-4,5-dione ; la 3-éthoxy pyrazolin-4,5-dione ; la 3-carboxy 1-méthyl pyrazolin-4,5-dione ; la 3-méthoxycarbonyle 1-méthyl pyrazolin-4,5-dione ; la 3-éthoxycarbonyle 1-méthyl pyrazolin-4,5-dione.

L'invention a également pour objet un premier procédé de préparation des nouveaux composés de formule (I'), ce procédé étant caractérisé par le fait qu'il comprend les étapes suivantes (les significations de R'₁ et R'₂ sont telles que données ci-avant) :
i) on fait réagir une pyrazolin-5-one ① avec un composé nitroso-aromatique ② de façon à obtenir la 4-arylimino pyrazolin-5-one correspondante ③ : 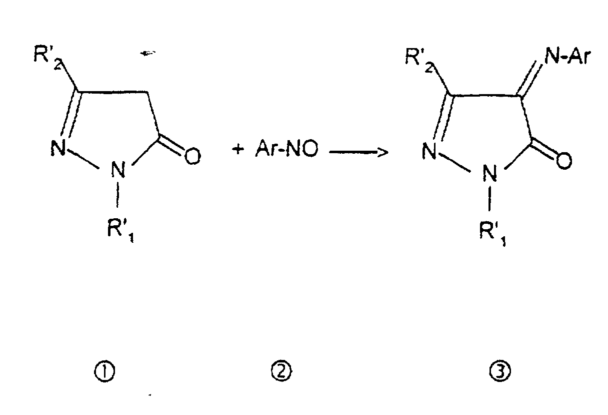 cette réaction étant conduite de préférence dans un alcool inférieur tel que le méthanol, l'éthanol ou l'isopropanol à une température comprise entre 65 °C et 85 °C, au reflux du solvant utilisé, et de préférence en présence d'une base faible de type carbonate ou bicarbonate en quantité catalytique,
ii) puis on hydrolyse la 4-arylimino pyrazolin-5-one ③ , de préférence en milieu acide fort, pour obtenir le dérivé de pyrazolin-4,5 dione correspondant de formule (l'):

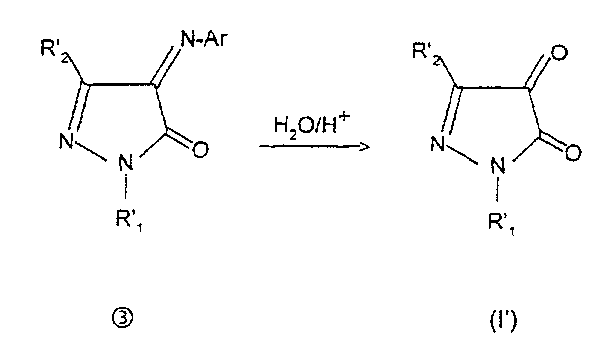

Dans un tel procédé, le dérivé nitroso aromatique de la première étape est de préférence une p-nitrosodialkylaniline de formule ② ' : 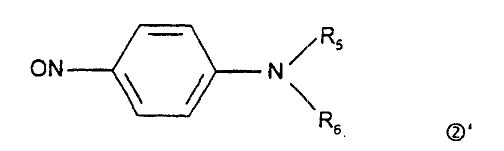 dans laquelle R₅ et R₆ représentent un radical alkyle en C₁-C₄, linéaire ou ramifié.

L'hydrolyse acide de la deuxième étape du procédé de préparation selon l'invention est réalisée de préférence avec de l'acide sulfurique dilué ou de l'acide chlorhydrique aqueux à température ambiante en présence d'un co-solvant de la pyrazolin-4,5-dione, non miscible à l'eau, ce qui permet d'extraire avantageusement le composé au fur et à mesure de sa formation facilitant son isolement avec une très grande pureté. Le co-solvant non miscible à l'eau peut être un solvant halogéné tel que par exemple le dichlorométhane ou le 1,2-dichloroéthane. Dans une forme préférée de réalisation de l'invention, le co-solvant non miscible à l'eau est un éther tel que l'éther diéthylique ou diisopropylique.

L'invention a également pour objet un deuxième procédé de préparation des nouveaux composés de formule (I'), ce procédé étant caractérisé par le fait qu'il comprend les étapes suivantes (les significations de R'₁ et R'₂ sont telles que données ci-avant) :
i) on fait réagir du brome sur une pyrazolin-5-one de formule ① pour obtenir la 4,4-dibromo pyrazolin-5-one correspondante de formule ④ (étape a),
ii) puis on fait réagir du diacétate de plomb de façon à former le diacétate correspondant de formule ⑤ , produit intermédiaire instable qui conduit spontanément, par élimination d'anhydride acétique, au dérivé de pyrazolin-4,5-dione de formule (I') désiré (étape b) , ces deux étapes a et b étant donc conduites selon le schéma réactionnel suivant :

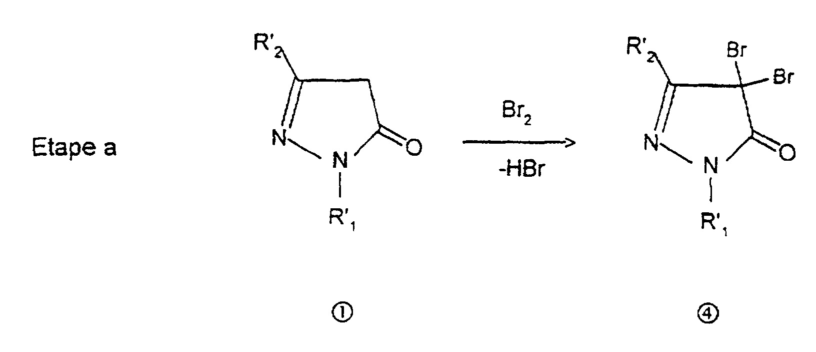

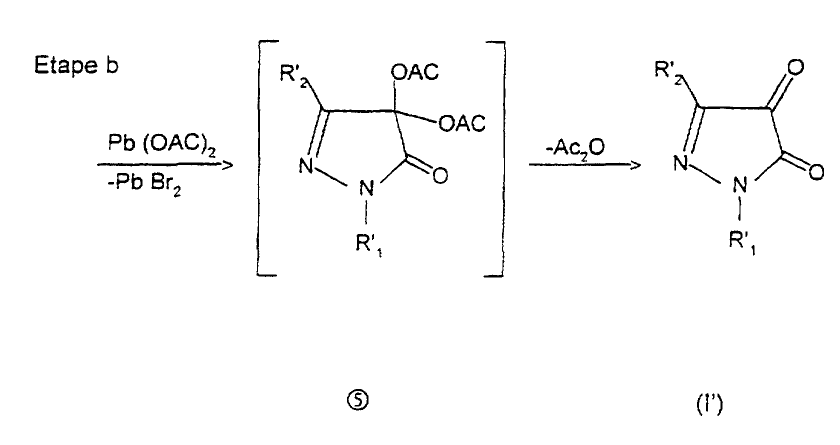

La première étape de dibromation est réalisée de préférence en milieu aqueux en présence de 2 équivalents de brome à température ambiante. La réaction est généralement complète en quelques heures : le dérivé dibromé précipite au fur et à mesure de sa formation, ce qui permet un isolement par simple filtration avec une grande pureté.

La deuxième étape est conduite avantageusement au reflux de l'acide acétique en quelques heures, le dibromure de plomb qui se forme pouvant être séparé très facilement par simple filtration.

Des exemples concrets, mais nullement limitatifs, destinés à illustrer l'invention vont maintenant être donnés.

### EXEMPLE 1 :

On a étudié, au travers d'un test comparatif in vivo, la coloration obtenue à partir de formules contenant la 3-méthyl-1-phényl-2-pyrazolin-4,5 dione d'une part, et la dihydroxyacétone (DHA) d'autre part.

On a ainsi réalisé trois formules A, B et C dont les composition sont rassemblées dans le tableau (I) suivant (les quantités sont exprimées en poids, par rapport au poids total de la composition) :

**Tableau (I) :**

| Composition (% en poids) | Formule A (invention) | Formule B (comparatif) | Formule C (comparatif) |
|---|---|---|---|
| 3-méthyl-1-phényl-2-pyrazolin-4,5-dione | 1 | 0 | 0 |
| Dihydroxyacétone | 0 | 1 | 5 |
| Excipient*qsp | 100 | 100 | 100 |

| | | | |
|---|---|---|---|
| (*) L'excipient est un mélange eau/éthanol/1,2-propane diol dans un rapport pondéral 1/2/1. | | | |

### Protocole d'évaluation :

Les colorations obtenues avec les formules décrites ci-dessus ont été évaluées au moyen d'un test in vivo sur trois volontaires selon la méthode suivante : on a appliqué sur la peau les formules A, B et C sur des zones de 2.5 x 2.5 cm délimitées sur les avant-bras des trois modèles. Puis on a ensuite déterminé l'intensité de coloration qui était attachée à chacune des formules après un certain temps T, soit ΔL_{T}. Pour ce faire, on a effectué des mesures colorimétriques à l'aide d'un colorimètre Minolta CM 1000. Une première mesure a été effectuée juste avant l'application des produits (T₀), une deuxième 30 minutes après l'application des produits (T₃₀ₘᵢₙ), et une dernière 5 heures après l'application des produits (T_{5H}).

Les résultats sont exprimés dans le système (L, a, b) dans lequel L représente la luminance, a représente l'axe rouge-vert (-a = vert. +a = rouge) et b représente l'axe jaune-bleu (-b =bleu, +b =jaune).

Pour l'évaluation de l'intensité de la coloration, on s'intéresse à ΔL (ΔL = L_{T}-L_{T0}) qui quantifie l'assombrissement de la couleur : plus ΔL est négatif, plus la couleur s'est assombrie.

Les résultats (valeurs moyennes de ΔL obtenues 30 mn et 5h après application) sont consignés dans le tableau (II) suivant

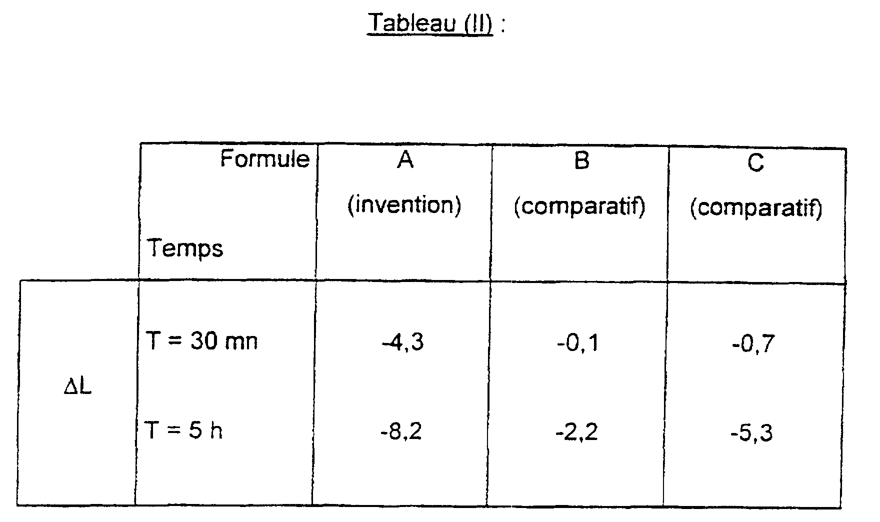

La composition A renfermant la 3-méthyl-1-phényl-2-pyrazolin-4,5-dione selon l'invention conduit à une coloration cutanée d'apparition plus rapide et d'intensité plus importante que les compositions B et C contenant la DHA (à teneur égale pour B ou 5 fois supérieure pour C à celle de la 3-méthyl-1-phényl-2-pyrazolin-4,5-dione).

### EXEMPLE 2 :

On donne ci-après un exemple concret d'une émulsion huile-dans-eau autobronzante conforme à l'invention :

### Phase A :

- mélange d'alcool cétylstéarylique et d'alcool cétylstéarylique oxyéthyléné (33 OE) 80/20 vendu sous la dénomination « DEHSCONET 390 » par la société TENSIA 7 %
- mélange de mono et distéarate de glycérol vendu sous la dénomination « CERASYNTH SD » par ta société ISP 2 %
- alcool cétylique 1,5 %
- polydiméthylsiloxane vendu sous la dénomination « DC200 Fluid » par la société DOW CORNING 1,5 %
- benzoate d'alcools C12/C15 vendu sous la dénomination « FINSOLV TN » par la société FINETEX 15 %
- 3-méthyl-1-phényl-2-pyrazolin-4,5-dione 1 %

### Phase B :

- glycérine 20 %
- conservateurs qs
- eau déminéralisée qsp 100 %

Cette émulsion a été réalisée selon le mode opératoire suivant : on a amené les phases A et B à 80 °C séparément. Puis on a versé la phase A dans la phase B sous agitation Moritz. On a ensuite homogénéisé le mélange puis on l'a laissé refroidir jusqu'à température ambiante.

### EXEMPLE 3 :

On donne ici des exemples concrets de compositions conformes à l'invention pour colorer les cheveux :

### Composition 1 :

- 3-méthyl 1-phényl pyrazolin-4,5-dione 0,113 g
- alcool benzylique 2 g
- alcool gras C₁₆-C₁₈ 1,8 g
- sodium éther sulfate d'alcool gras C₁₆-C₁₈ 1,8 g
- eau 14 g

Le pH de la composition a été ajusté à une valeur de 2,6 avec de l'acide chlorhydrique à 10 %.

Cette composition a été appliquée pendant 30 minutes sur des mèches de cheveux gris naturels à 90 % de blancs et sur des mèches de cheveux gris permanentés à 90 % de blancs, à raison de 2 à 5 g/g de cheveux. La composition a été appliquée soit à température ambiante (20 °C), soit à 50°C.

Les mèches ont ensuite été rincées, soit abondamment à l'eau du robinet, soit à l'aide d'un shampooing standard. Elles ont ensuite été séchées pendant 10 minutes, soit au sèche-cheveux, soit sous casque à une température comprise entre 60 et 65 °C.

On a ensuite déterminé l'intensité de coloration des mèches. Les résultats sont donnés dans le système (L, a, b) dans lequel L représente la luminance, a représente l'axe rouge-vert (-a = vert, +a = rouge) et b représente l'axe jaune-bleu (-b =bleu, +b =jaune).

Plus a est élevé, plus la mèche est rouge.

Les résultats pour les cheveux avant traitement (non traités) et après traitement par la composition 1 conforme à l'invention (traités) sont rassemblés dans le tableau (III) suivant :

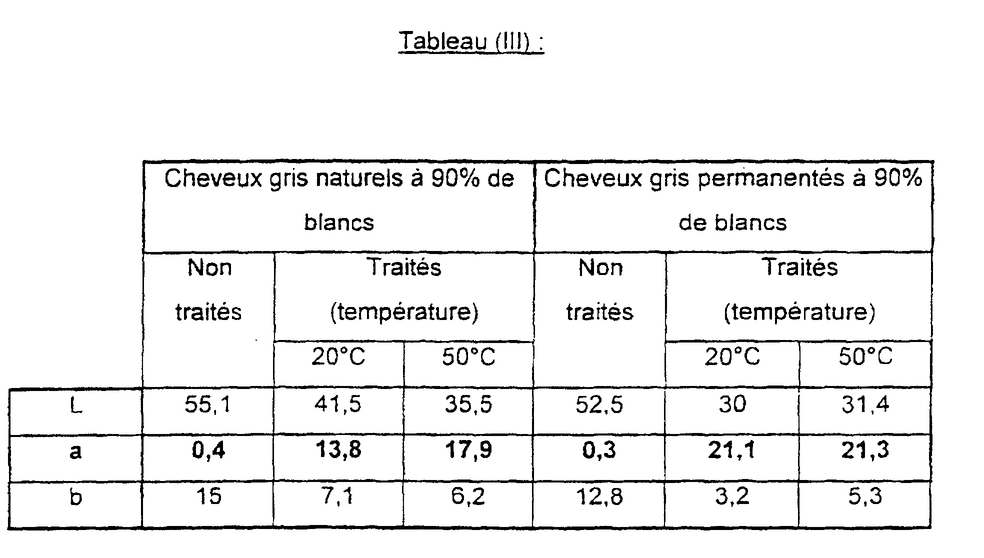

Ces résultats montrent clairement que les mèches ont été colorées en rouge intense.

### Composition 2 :

- 3-méthyl 1-phényl pyrazolin-4,5-dione 0,113 g
- alcool benzylique 2 g
- alcool gras C₁₆-C₁₈ 1,8 g
- sodium éther sulfate d'alcool gras C₁₆-C₁₈ 1,8 g
- eau 14 g

Le pH de la composition a été ajusté à une valeur de 5,8 avec de la triéthanolamine.

La composition a été appliquée 30 minutes sur des mèches de cheveux de mêmes natures que ci-dessus (cheveux gris naturels à 90 % de blancs et sur des mèches de cheveux gris permanentés à 90 % de blancs), à raison de 2 à 5 g/g à une température de 37°C.

Les mèches ont ensuite été rincées et séchées comme ci-dessus. L'intensité de leur coloration a également été mesurée comme ci-dessus.

Les résultats pour les cheveux avant traitement (non traités) et après traitement par la composition 2 conforme à l'invention (traités) sont rassemblés dans le tableau (IV) suivant :

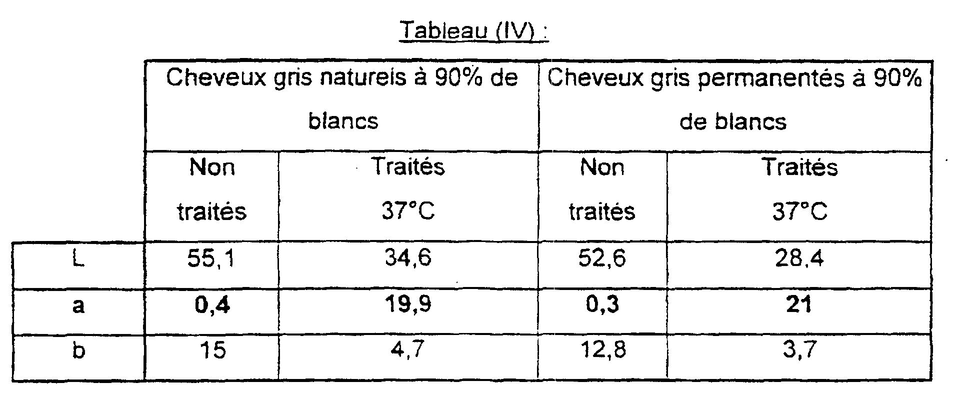

Ces résultats montrent clairement que les mèches ont été colorées en rouge intense.

## Revendications

1. Composition comprenant, dans un support cosmétiquement acceptable, au moins une pyrazolin-4,5 dione répondant à la formule (I) ci-dessous : 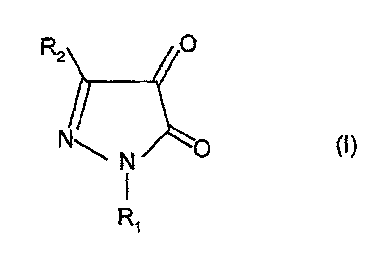 dans laquelle R₁ est choisi parmi :
- un atome d'hydrogène ; un radical alkyle, linéaire ou ramifié, en C₁-C₁₈ éventuellement substitué par un radical hydroxyle (OH), sulfonyle (SO₃H) ou carboxyle (COOH); un radical cyclohexyle ou cyclopentyle;
- un radical 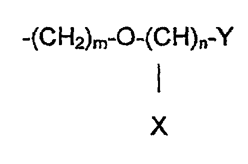 dans lequel m est égal à 1, 2 ou 3, n est égal à 1, 2 ou 3, X est un atome d'hydrogène ou un radical méthyle, et Y est un radical méthyle, hydroxy ou alcoxy en C₁-C₅, linéaire ou ramifié ;
- un radical -(CH₂)ₚ -OR' dans lequel R' représente un radical phényle ou naphtyle et p est égal à 1 ou 2 ;
- un radical -(CH₂)_{q}-R" dans lequel q est égal à 1,2 ou 3 et R" est choisi parmi :
- un radical phényle substitué ou non par au maximum 2 radicaux choisis parmi les radicaux méthyle, trifluorométhyle, méthoxy ou sulfonyle ;
- un radical naphtyle, un radical thiényle, un radical furyle, un radical pyridyle ou un radical pipéridyle ;
- un radical : 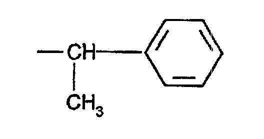
- un radical : 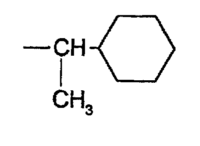
- un radical : 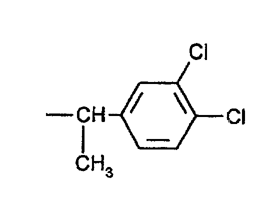
- un radical phényle ; un radical phényle substitué par un radical nitro ; un radical phényle substitué par un à trois radicaux choisis parmi : -COOH, -CH₂COOH, -Cl, -Br, -F, -SO₃H, -CH₂OH, -OCF₃, -CF₃, -SO₂CH₃, -SO₂NH₂, -SO₂NHC₂H₅,-SO₂NHCH₂CH₂OH, -CON(CH₃)₂, -CON(C₂H₅)₂, -CH₂N(CH₃)₂, -CH₂N(C₂H₅)₂, -NHCOCH₃, -NHCOC₂H₅, un radical alkyle en C₁-C₈ ou -ZR₃ dans lequel Z est un atome d'oxygène ou de soufré et R₃ est un atome d'hydrogène ou un radical alkyle linéaire ou ramifié en C₁-C₁₈;
- un radical naphtyle éventuellement substitué par un radical -SO₃H ;
- un radical benzyle substitué par un radical -COOH ;
- un radical pyridyle, un radical pyrimidinyle, un radical pyrazinyle, un radical triazinyle, un radical benzotriazolyle, un radical benzimidazolyle, un radical thiényle, un radical imidazolyle, un radical thiazolyle, un radical triazolyle-1,2,4, un radical indazolyle, un radical indolyle, un radical quinolyle ou un radical isoquinolyle ;
et R₂ est choisi parmi :
- un atome d'hydrogène ; un radical alkyle en C₁-C₆, linéaire ou ramifié ; un radical hydroxyalkyle en C₁-C₄ ;
- un radical phényle ; un radical phényle substitué par un atome d'halogène, un radical nitro ou un radical trifluorométhyle ; un radical phényle substitué par au maximum trois radicaux choisis parmi les radicaux alkyle en C₁-C₄, alcoxy en C₁-C₄, dialkylamino en C₁-C₄ ou alkylthio en C₁-C₂;
- un radical benzyle ; un radical benzyle substitué par un atome d'halogène, un radical alkyle en C₁-C₄, un radical alcoxy en C₁-C₄, un radical trifluorométhyle ou un radical dialkylamino en C₁-C₄ ;
- un radical -(CH₂)ᵣ-R₄ dans lequel r est égal à 1,2 ou 3 et R₄ est choisi parmi :
- un radical -SO₃H, un radical akylthio en C₁-C₂ ou un radical benzylthio ;
- un radical méthoxycarbonyle ou éthoxycarbonyle ;
- un radical alcoxy en C₁-C₄ ou un radical phénoxy éventuellement substitué par un ou plusieurs atomes d'halogène;
- un radical alcoxy en C₁-C₄; un radical trifluorométhyle ; acétamido ; dialkylamino en C1-C4 ; carboxy ; méthoxycarbonyle ; éthoxycarbonyle ;
- un radical thiényle, un radical furyle, un radical pyridyle ou un radical pyrazolyle.

2. Composition selon la revendication 1, caractérisée par le fait que R₁ est choisi parmi:
- un atome d'hydrogène ; un radical alkyle, linéaire ou ramifié, en C₁-C₈,
- un radical -(CH₂)₂-OR' dans lequel R' représente un radical phényle ou naphtyle ;
- un radical -(CH₂)_{q}-R" dans lequel q est égal à 1 ou 2 et R" représente un radical phényle éventuellement substitué par un radical trifluorométhyle ;
- un radical phényle éventuellement substitué par un radical nitro, un radical -Cl, un radical alkyle en C1-C4 ou un radical alcoxy en C1-C4.

3. Composition selon la revendication 1 ou 2, caractérisée par le fait que R₂ est choisi parmi
- un atome d'hydrogène ; un radical alkyle en C₁-C₄, linéaire ou ramifié ; un radical hydroxyalkyle en C₁-C₄; un radical méthoxyméthyle ;
- un radical phényle éventuellement substitué par un atome d'halogène, un radical nitro, un radical alkyle en C₁-C₄ ou un radical alcoxy en C₁-C₄ :
- un radical alcoxy en C₁-C₄ ; un radical trifluorométhyle ; acétamido ; dialkylamino en C1-C4 ; carboxy ; méthoxycarbonyle ; éthoxycarbonyle ;
- un radical thiényle, un radical furyle, un radical pyridyle ou un radical pyrazolyle.

4. Composition selon la revendication 3, caractérisée par le fait que R₁ est choisi parmi:
- l'hydrogène et les radicaux méthyle, éthyle, isopropyle, tertiobutyle ou phényle;
et R₂ est choisi parmi :
- l'hydrogène ou les radicaux méthyle, phényle, méthoxyphényle, méthoxy, éthoxy, carboxy, méthoxycarbonyle, éthoxycarbonyle, acétamido, diméthylamino, diéthyiamino, trifluorométhyle, furyle, pyridyle.

5. Composition selon la revendication 4, caractérisée par le fait que la pyrazolin-4,5 dione est choisie parmi : la 3-méthyl 1-phényl pyrazolin-4,5-dione ; la 3-méthyl pyrazolin-4,5-dione ; la 1,3-diméthyl pyrazolin-4,5-dione ; la 1-éthyl 3-méthyl pyrazolin-4,5-dione ; la 1-isopropyl 3-méthyl pyrazolin-4,5-dione; la 1-tertbutyl 3-méthyl pyrazolin-4,5-dione; la 1-méthyl 3-phényl pyrazolin-4,5-dione ; la 1-méthyl 3-(3'-méthoxyphényl) pyrazolin-4,5-dione; la 3(2'-furyl) 1-méthyl pyrazolin-4,5-dione ; la 1-méthyl 3-(4'-pyridyl) pyrazolin-4,5-dione ; la 1-méthyl 3-méthoxy pyrazolin-4,5-dione ; la 3-éthoxy 1-méthyl pyrazolin-4,5-dione ; la 3-diméthylamino 1-méthyl pyrazolin-4,5-dione : la 3-diéthylamino 1-méthyl pyrazolin-4,5-dione; la 3-acétamido 1-méthyl pyrazolin-4,5-dione; la 1-phényl pyrazolin-4,5-dione; la 1-méthyl pyrazolin-4,5-dione ; la 1-éthyl pyrazolin-4,5-dione ; la 1-isopropyl pyrazolin-4,5-dione; la 1-tertbutyl pyrazolin-4,5-dione ; la 3-méthoxy 1-phényl pyrazolin-4,5-dione ; la 3-éthoxy 1-phényl pyrazolin-4,5-dione ; la 3-acétamido 1-phényl pyrazolin-4,5-dione ; la 3-diméthylamino 1-phényl pyrazolin-4,5-dione ; la 3-diéthylamino 1-phényl pyrazolin-4,5-dione ; la 1-phényl 3-trifluorométhyl pyrazolin-4,5-dione; la 1-méthyl 3-trifluorométhyl pyrazolin-4,5-dione ; la 1-isopropyl 3-trifluorométhyl pyrazolin-4,5-dione; la 1-éthyl 3-trifluorométhyl pyrazolin-4,5-dione ; la 3-trifluorométhyl pyrazolin-4,5-dione ; la 3-carboxy 1-phényl pyrazolin-4,5-dione ; la 3-méthoxycarbonyl 1-phényl pyrazolin-4,5-dione ; la 3-éthoxycarbonyl 1-phényl pyrazolin-4,5-dione ; la 3-méthoxy pyrazolin-4,5-dione ; la 3-éthoxy pyrazolin-4,5-dione ; la 3-carboxy 1-méthyl pyrazolin-4,5-dione; la 3-méthoxycarbonyle 1-méthyl pyrazolin-4,5-dione ; la 3-éthoxycarbonyle 1-méthyl pyrazolin-4,5-dione.

6. Composition selon l'une quelconque des revendications 1 à 5, caractérisée par le fait que la pyrazolin-4,5 dione est présente dans la composition à une concentration comprise entre 0,05 et 10 % en poids, par rapport au poids total de la composition.

7. Composition selon la revendication 6, caractérisée par le fait que ladite concentration est comprise entre 0,05 et 5 % en poids, par rapport au poids total de la composition.

8. Composition selon l'une quelconque des revendications précédentes, caractérisée par le fait qu'il s'agit d'une composition cosmétique pour colorer la peau.

9. Composition selon l'une quelconque des revendications 1 à 7, caractérisée par le fait qu'il s'agit d'une composition cosmétique pour colorer les cheveux.

10. Procédé de traitement cosmétique de la peau destiné à la bronzer et/ou la brunir artificiellement, caractérisé par le fait qu'il consiste à appliquer sur celle-ci une quantité efficace d'une composition telle que définie à l'une quelconque des revendications 1 à 8, ou d'une pyrazolin-4,5-dione telle que définie à l'une quelconque des revendications 1 à 5.

11. Utilisation d'une pyrazolin-4,5-dione telle que définie à l'une quelconque des revendications 1 à 5 dans, ou pour la fabrication de, compositions destinées au bronzage et/ou au brunissage artificiels de la peau.

12. Procédé de traitement cosmétique des cheveux destiné à les colorer, caractérisé par le fait qu'il consiste à appliquer sur les cheveux une quantité efficace d'une composition telle que définie à l'une quelconque des revendications 1 à 9 ou d'une pyrazolin-4,5-dione telle que définie à l'une quelconque des revendications 1 à 5.

13. Utilisation d'une pyrazolin-4,5-dione telle que définie à l'une quelconque des revendications 1 à 5 dans, ou pour la fabrication de, compositions destinées à colorer les cheveux.

14. Composés répondant à la formule générale (I') suivante : 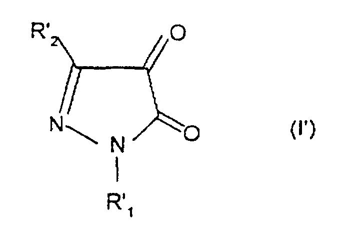 dans laquelle R'₁ et R'₂ ont les mêmes significations que celles indiquées dans la formule (I) de la revendication 1 pour R₁ et R₂ avec néanmoins les réserves suivantes :
- (i) lorsque R'₁ représente un radical phényle, alors R'₂ est différent d'un atome d'hydrogène, d'un radical méthyle, d'un radical tertiobutyle, d'un radical phényle, d'un radical p-méthylphényle, d'un radical p-nitrophényle ou d'un radical p-méthoxyphényle,
- (ii) lorsque R'₂ représente un radical méthyle, R'₁ est différent d'un radical p-nitrophényle, d'un radical 2-phénoxyéthyle, d'un radical 2-naphtyloxyéthyle, d'un radical méthyle, d'un radical m-trifluorométhylbenzyle, d'un radical 1-phényléthyle, d'un radical p-méthylphényle, d'un radical 1-cyclohexyléthyle ou d'un radical 1-(3',4'-dichlorophényl) éthyle,
- (iii) lorsque R'₁ représente un radical 2-naphtyloxyéthyle, R'₂ est différent d'un radical n-propyle ou d'un radical hydroxyméthyle,
- (iv) lorsque R'₁ représente un radical phénoxy-2 éthyle, R'₂ est différent d'un radical tertiobutyle,
- (v) lorsque R'₂ représente un radical méthoxyméthyle, R'₁ est différent d'un radical 2-naphtyloxyéthyle ou d'un radical 1-(3',4'-dichlorophényl) éthyle,
- (vi) lorsque R'₁ représente un atome d'hydrogène, R'₂ est différent d'un radical méthyle,
- (vii) lorsque R'₂ représente un radical phényle, R'₁ est différent d'un radical méthyle, d'un radical p-méthoxyphényle ou d'un radical p-nitrophényle.

15. Composés selon la revendication 14, caractérisés par le fait que R'₁ est choisi parmi:
- un atome d'hydrogène ; un radical alkyle, linéaire ou ramifié, en C1-C8,
- un radical -(CH2)₂-OR' dans lequel R' représente un radical phényle ou naphtyle ;
- un radical -(CH2)q-R" dans lequel q est égal à 1 ou 2 et R" représente un radical phényle éventuellement substitué par un radical trifluorométhyle ;
- un radical phényle éventuellement substitué par un radical nitro, un radical -Cl, un radical alkyle en C1-C4 ou un radical alcoxy en C1-C4.

16. Composés selon la revendication 14 ou 15, caractérisés par le fait que R'₂ est choisi parmi :
- un atome d'hydrogène : un radical alkyle en C1-C4, linéaire ou ramifié ; un radical hydroxyalkyle en C1-C4 ; un radical méthoxyméthyle ;
- un radical phényle éventuellement substitué par un atome d'halogène, un radical nitro, un radical alkyle en C1-C4 ou un radical alcoxy en C1-C4 ;
- un radical alcoxy en C1-C4 ; un radical trifluorométhyle ; acétamido ; dialkylamino en C1-C4 ; carboxy ; méthoxycarbonyle ; éthoxycarbonyle ;
- un radical thiényle, un radical furyle, un radical pyridyle ou un radical pyrazolyle.

17. Composés selon la revendication 16, caractérisés par le fait que R'₁ est choisi parmi:
- l'hydrogène ou les radicaux méthyle, éthyle, isopropyle, tertiobutyle ou phényle;
et R'₂ est choisi parmi :
- l'hydrogène ou les radicaux méthyle, phényle, méthoxyphényle, méthoxy, éthoxy, carboxy, méthoxycarbonyle, éthoxycarbonyle, acétamido, diméthylamino, diéthylamino, trifluorométhyle, furyle, pyridyle.

18. Composé selon la revendication 17, caractérisé par le fait qu'il est choisi parmi ; la 1-éthyl 3-méthyl pyrazolin-4,5-dione ; la 1-isopropyl 3-méthyl pyrazolin-4,5-dione ; la 1-tertbutyl 3-méthyl pyrazolin-4,5-dione ; la 1-méthyl 3-(3'-méthoxyphényl) pyrazolin-4,5-dione; la 3(2'-furyl) 1-méthyl pyrazolin-4,5-dione ; la 1-méthyl 3-(4'-pyridyl) pyrazolin-4,5-dione ; la 1-méthyl 3-méthoxy pyrazolin-4,5-dione ; la 3-éthoxy 1-méthyl pyrazolin-4,5-dione ; la 3-diméthylamino 1-méthyl pyrazolin-4,5-dione; la 3-diéthylamino 1-méthyl pyrazolin-4,5-dione ; la 3-acétamido 1-méthyl pyrazolin-4,5-dione ; la 1-méthyl pyrazolin-4,5-dione ; la 1-éthyl pyrazolin-4,5-dione; la 1-isopropyl pyrazolin-4,5-dione; la 1-tertbutyl pyrazolin-4,5-dione ; la 3-méthoxy 1-phényl pyrazolin-4,5-dione ; la 3-éthoxy 1-phényl pyrazolin-4,5-dione ; la 3-acétamido 1-phényl pyrazolin-4,5-dione ; la 3-diméthylamino 1-phényl pyrazolin-4,5-dione; la 3-diéthylamino 1-phényl pyrazolin-4,5-dione ; la 1-phényl 3-trifluorométhyl pyrazolin-4,5-dione ; la 1-méthyl 3-trifluorométhyl pyrazolin-4,5-dione ; la 1-isopropyl 3-trifluorométhyl pyrazolin-4,5-dione ; la 1-éthyl 3-trifluorométhyl pyrazolin-4,5-dione ; la 3-trifluorométhyl pyrazolin-4,5-dione; la 3-carboxy 1-phényl pyrazolin-4,5-dione; la 3-méthoxycarbonyl 1-phényl pyrazolin-4,5-dione; la 3-éthoxycarbonyl 1-phényl pyrazolin-4,5-dione; la 3-méthoxy pyrazolin-4,5-dione ; la 3-éthoxy pyrazolin-4,5-dione ; la 3-carboxy 1-méthyl pyrazolin-4,5-dione; la 3-méthoxycarbonyle 1-méthyl pyrazolin-4,5-dione ; la 3-éthoxycarbonyle 1-méthyl pyrazolin-4,5-dione.

19. Procédé de préparation des nouveaux composés de formule (I'), caractérisé par le fait qu'il comprend les étapes suivantes, les significations de R'₁ et R'₂ étant telles que définies à la revendication 14 :
i) on fait réagir une pyrazolin-5-one ① avec un composé nitroso-aromatique ② pour obtenir la 4-arylimino pyrazolin-5-one correspondante ③ : 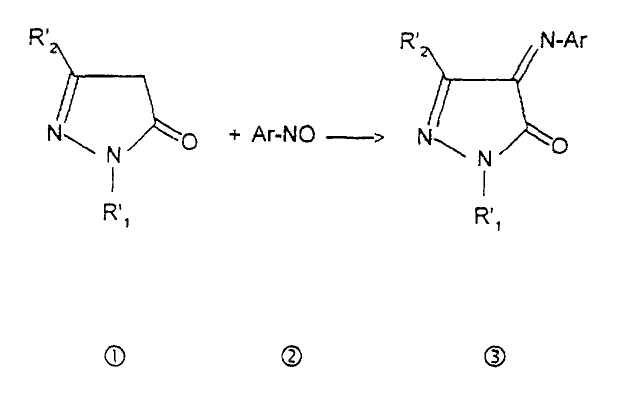 cette réaction étant conduite dans un alcool inférieur, au reflux, à une température comprise entre 65 °C et 85 °C,
ii) puis on hydrolyse la 4-arylimino pyrazolin-5-one ③ , en milieu acide fort, pour obtenir le dérivé de pyrazolin-4,5 dione correspondant de formule (I') :

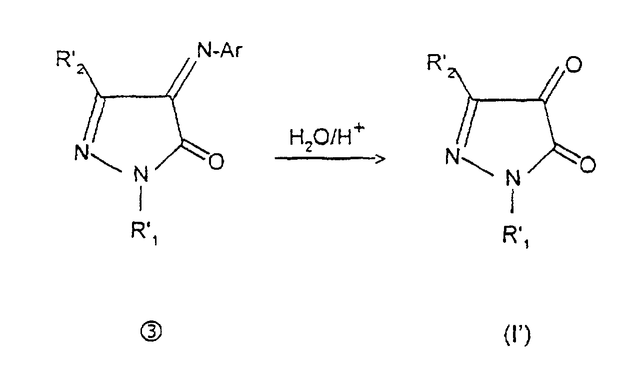

20. Procédé selon la revendication 19, caractérisé par le fait que l'alcooi inférieur utilisé à l'étape (i) est choisi parmi le méthanol, l'éthanol ou l'isopropanol.

21. Procédé selon la revendication 19 ou 20, caractérisé par le fait que l'étape (i) s'effectue en présence d'une base faible telle qu'un carbonate ou un bicarbonate en quantité catalytique.

22. Procédé selon l'une quelconque des revendications 19 à 21, caractérisé par le fait que le dérivé nitroso aromatique de l'étape (i) est une p-nitrosodialkylaniline de formule ② ' suivante : 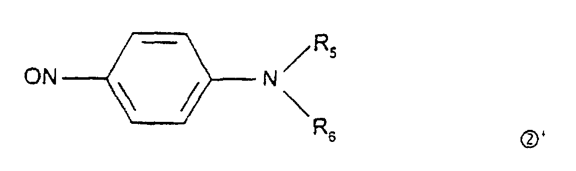 dans laquelle R₅ et R₆ représentent un radical alkyle en C₁-C₄, linéaire ou ramifié.

23. Procédé selon l'une quelconque des revendications 19 à 22, caractérisé par le fait que l'hydrolyse acide de l'étape (ii) est réalisée avec de l'acide sulfurique dilué ou de l'acide chlorhydrique aqueux à température ambiante en présence d'un co-solvant de la pyrazolin-4,5-dione non miscible à l'eau.

24. Procédé selon la revendication 23, caractérisé par le fait que ledit co-solvant est un éther choisi parmi l'éther diéthylique et l'éther diisopropylique.

25. Procédé de préparation des nouveaux composés de formule (I'). caractérisé par le fait qu'il comprend les étapes suivantes, les significations de R'₁ et R'₂ étant telles que définies à la revendication 14 :
i) on fait réagir du brome sur une pyrazolin-5-one de formule ① pour obtenir la 4,4-dibromo pyrazolin-5-one correspondante de formule ④ (étape a),
ii) puis on fait réagir du diacétate de plomb de façon à former le diacétate correspondant de formule ⑤ , produit intermédiaire instable qui conduit spontanément, par élimination d'anhydride acétique, au dérivé de pyrazolin-4,5-dione de formule (I') (étape b), ces deux étapes étant conduites selon le schéma réactionnel suivant :

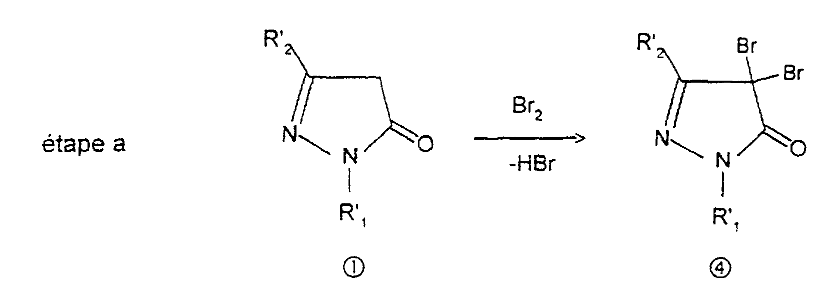

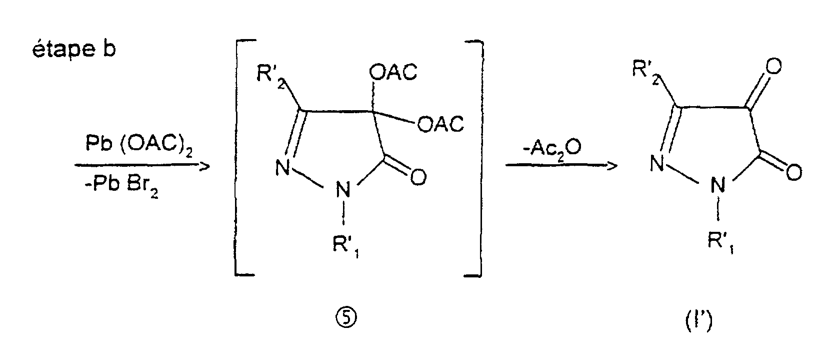

26. Procédé selon la revendication 25, caractérisé par le fait que l'étape a est réalisée en milieu aqueux en présence de 2 équivalents de brome à température ambiante.

27. Procédé selon la revendication 25 ou 26, caractérisé par le fait que l'étape b est conduite au reflux de l'acide acétique pendant quelques heures.

## Patentansprüche

1. Zusammensetzung, die in einem kosmetisch akzeptablen Träger mindestens ein Pyrazolin-4,5-dion der folgenden Formel (I) 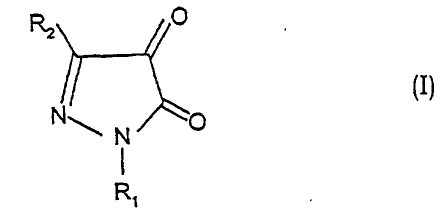 enthält, in der R₁ ausgewählt ist unter
- Wasserstoff, geradkettigem oder verzweigtem C₁-C₁₈-Alkyl, das gegebenenfalls mit einer Hydroxygruppe (OH), Sulfonylgruppe (SO₃H) oder Carboxygruppe (COOH) substituiert ist, Cyclohexyl und Cyclopentyl,
- dem Rest 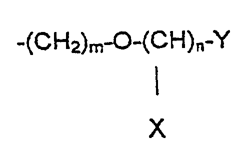 worin bedeuten: m die Zahl 1, 2 oder 3, n die Zahl 1, 2 oder 3, X Wasserstoff oder Methyl und Y Methyl, eine Hydroxygruppe oder einen geradkettigen oder verzweigten C₁-C₅-Alkoxyrest,
- dem Rest -(CH₂)ₚ-OR', worin R' Phenyl oder Naphthyl bedeutet und p die Zahl 1 oder 2 ist,
- dem Rest -(CH₂)_{q}-OR", worin q die Zahl 1, 2 oder 3 bedeutet und R" ausgewählt ist unter
• Phenyl, das nicht substituiert ist oder mit maximal 2 Resten substituiert ist, die unter Methyl, Trifluormethyl, Methoxy und Sulfonyl ausgewählt sind,
• Naphthyl, Thienyl, Furyl, Pyridyl und Piperidyl,
- dem Rest 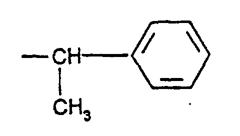
- dem Rest 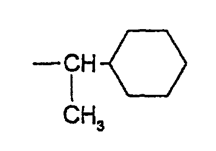
- dem Rest, 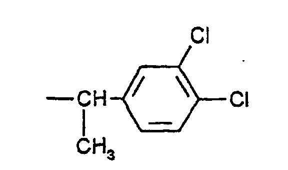
- nicht substituiertem Phenyl, Phenyl, das mit einer Nitrogruppe substituiert ist, Phenyl, das mit ein bis drei Resten substituiert ist, die ausgewählt sind unter -COOH, CH₂COOH, -Cl, -Br, -F, -SO₃H, -CH₂OH, -OCF₃, -CF₃, -SO₂CH₃, -SO₂NH₂, -SO₂NHC₂H₅; -SO₂NHCH₂CH₂OH, -CON(CH₃)₂, -CON(C₂H₅)₂, -CH₂N(CH₃)₂, -CH₂N(C₂H₅)₂, -NHCOCH₃, -NHCOC₂H₅, C₁-C₈-Alkyl und ZR₃, worin Z ein Sauerstoffatom oder ein Schwefelatom darstellt und R₃ ein Wasserstoffatom oder geradkettiges oder verzweigtes C₁-C₁₈-Alkyl bedeutet,
- Naphthyl, das gegebenenfalls mit einer Gruppe -SO₃H substituiert ist,
- Benzyl, das mit einer Gruppe -COOH substituiert ist,
- Pyridyl, Pyrimidinyl, Pyrazinyl, Triazinyl, Benzotriazolyl, Benzimidazolyl, Thienyl, Imidazolyl, Thiazolyl, 1,2,4-Triazolyl, Indazolyl, Indolyl, Chinolyl und Isochinolyl,
und in der R₂ ausgewählt ist unter
- Wasserstoff, geradkettigem oder verzweigtem C₁-C₆-Alkyl, C₁-C₄-Hydroxyalkyl,
- nicht substituiertem Phenyl, Phenyl, das mit einem Halogenatom, einer Nitrogruppe oder einer Trifluormethylgruppe substituiert ist, Phenyl, das mit maximal drei Resten substituiert ist, die unter C₁-C₄-Alkyl, C₁-C₄-Alkoxy, C₁-C₄-Dialkylamino und C₁-C₂-Alkylthio ausgewählt sind,
- nicht substituiertem Benzyl, Benzyl, das mit einem Halogenatom C₁-C₄-Alkyl, C₁-C₄-Alkoxy, Trifluormethyl oder C₁-C₄-Dialkylamino substituiert ist,
- dem Rest -(CH₂)ᵣ-R₄, worin R die Zahl 1, 2 oder 3 bedeutet und R₄ ausgewählt ist unter:
• der Gruppe -SO₃H, C₁-C₂-Alkylthio und Benzylthio,
• Methoxycarbonyl und Ethoxycarbonyl,
• C₁-C₄-Alkoxy und Phenoxy, das gegebenenfalls mit einem oder mehreren Halogenatomen substituiert ist,
- C₁-C₄-Alkoxy, Trifluormethyl, Acetamido, C₁-C₄-Dialkylamino, Carboxy, Methoxycarbonyl, Ethoxycarbonyl,
- Thienyl, Furyl, Pyridyl und Pyrazolyl.

2. Zusammensetzung nach Anspruch 1, dadurch gekennzeichnet, daß R₁ ausgewählt ist unter
- Wasserstoff, geradkettigem oder verzweigtem C₁-C₈-Alkyl,
- dem Rest -(CH₂)₂-OR', worin R Phenyl oder Naphthyl bedeutet,
- dem Rest -(CH₂)_{q}-R'', worin q die Zahl 1 oder 2 ist und R" Phenyl bedeutet, das gegebenenfalls mit einer Trifluormethylgruppe substituiert ist,
- Phenyl, das gegebenenfalls mit einer Nitrogruppe, Chlor, C₁-C₄-Alkyl oder C₁-C₄-Alkoxy substituiert ist.

3. Zusammensetzung nach Anspruch 1 oder 2, dadurch gekennzeichnet, daß R₂ ausgewählt ist unter
- Wasserstoff, geradkettigem oder verzweigtem C₁-C₄-Alkyl, C₁-C₄-Hydroxyalkyl, Methoxymethyl,
- Phenyl, das gegebenenfalls mit einem Halogenatom, einer Nitrogruppe, C₁-C₄-Alkyl oder C₁-C₄-Alkoxy substituiert ist,
- C₁-C₄-Alkoxy, Trifluormethyl, Acetamido, C₁-C₄-Dialkylamino, Carboxy, Methoxycarbonyl, Ethoxycarbonyl,
- Thienyl, Furyl, Pyridyl und Pyrazolyl.

4. Zusammensetzung nach Anspruch 3, dadurch gekennzeichnet, daß R₁ unter Wasserstoff, Methyl, Ethyl, Isopropyl, *tert*.-Butyl und Phenyl ausgewählt ist und daß R₂ unter Wasserstoff, Methyl, Phenyl, Methoxyphenyl, Methoxy, Ethoxy, Carboxy, Methoxycarbonyl, Ethoxycarbonyl, Acetamido, Dimethylamino, Diethylamino, Trifluormethyl, Furyl, Pyridyl ausgewählt ist.

5. Zusammensetzung nach Anspruch 4, dadurch gekennzeichnet, daß das Pyrazolin-4,5-dion ausgewählt ist unter: 3-Methyl-1-phenylpyrazolin-4,5-dion, 3-Methylpyrazolin-4,5-dion, 1,3-Dimethylpyrazolin-4,5-dion, 1-Ethyl-3-methylpyrazolin-4,5-dion, 1-Isopropyl-3-methylpyrazolin-4,5-dion, 1-*tert*.-Butyl-3-methylpyrazolin-4,5-dion, 1-Methyl-3-phenylpyrazolin-4,5-dion, 1-Methyl-3-(3'-methoxyphenyl)-pyrazolin-4,5-dion, 3-(2'-Furyl)-1-methylpyrazolin-4,5-dion, 1-Methyl-3-(4'-pyridyl)-pyrazolin-4,5-dion, 1-Methyl-3-methoxypyrazolin-4,5-dion, 3-Ethoxy-1-methylpyrazolin-4,5-dion, 3-Dimethylamino-1-methylpyrazolin-4,5-dion, 3-Diethylamino-1-methylpyrazolin-4,5-dion, 3-Acetamido-1-methylpyrazolin-4,5-dion, 1-Phenylpyrazolin-4,5-dion, 1-Methylpyrazolin-4,5-dion, 1-Ethylpyrazolin-4,5-dion, 1-Isopropylpyrazolin-4,5-dion, 1-*tert*.-Butylpyrazolin-4,5-dion, 3-Methoxy-1-phenylpyrazolin-4,5-dion, 3-Ethoxy-1-phenylpyrazolin-4,5-dion, 3-Acetamido-1-phenylpyrazolin-4,5-dion; 3-Dimethylamino-1-phenylpyrazolin-4,5-dion, 3-Diethylamino-1-phenylpyrazolin-4,5-dion, 1-Phenyl-3-trifluormethylpyrazolin-4,5-dion, 1-Methyl-3-trifluormethylpyrazolin-4,5-dion, 1-Isopropyl-3-trifluormethylpyrazolin-4,5-dion, 1-Ethyl-3-trifluormethylpyrazolin-4,5-dion, 3-Trifluormethylpyrazolin-4,5-dion, 3-Carboxy-1-phenylpyrazolin-4,5-dion, 3-Methoxycarbonyl-1-phenylpyrazolin-4,5-dion, 3-Ethoxycarbonyl-1-phenylpyrazolin-4,5-dion, 3-Methoxypyrazolin-4,5-dion, 3-Ethoxypyrazolin-4,5-dion, 3-Carboxy-1-methylpyrazolin-4,5-dion, 3-Methoxycarbonyl-1-methylpyrazolin-4,5-dion, 3-Ethoxycarbonyl-1-methylpyrazolin-4,5-dion.

6. Zusammensetzung nach einem der Ansprüche 1 bis 5, dadurch gekennzeichnet, daß das Pyrazolin-4,5-dion in der Zusammensetzung in einer Konzentration von 0,05 bis 10 Gew.-%, bezogen auf das Gesamtgewicht der Zusammensetzung, enthalten ist.

7. Zusammensetzung nach Anspruch 6, dadurch gekennzeichnet, daß diese Konzentration im Bereich von 0,05 bis 5 Gew.-%, bezogen auf das Gesamtgewicht der Zusammensetzung, liegt.

8. Zusammensetzung nach einem der vorhergehenden Ansprüche, dadurch gekennzeichnet, daß es sich um eine kosmetische Zusammensetzung zum Färben der Haut handelt.

9. Zusammensetzung nach einem der Ansprüche 1 bis 7, dadurch gekennzeichnet, daß es sich um eine kosmetische Zusammensetzung zum Färben der Haare handelt.

10. Verfahren zur kosmetischen Behandlung der Haut, das dazu dient, die Haut künstlich braun zu färben und/oder künstlich zu bräunen, dadurch gekennzeichnet, daß es darin besteht, auf die Haut eine wirksame Menge einer wie in einem der Ansprüche 1 bis 8 definierten Zusammensetzung oder eines wie in einem der Ansprüche 1 bis 5 definierten Pyrazolin-4,5-dions aufzutragen.

11. Verwendung eines wie in einem der Ansprüche 1 bis 5 definierten Pyrazolin-4,5-dions in oder für die Herstellung von Zusammensetzungen, die für die künstliche Braunfärbung und/oder künstliche Bräunung der Haut vorgesehen sind.

12. Verfahren zur kosmetischen Behandlung der Haare, das dazu dient, die Haare zu färben, dadurch gekennzeichnet, daß es darin besteht, auf die Haare eine wirksame Menge einer wie in einem der Ansprüche 1 bis 9 definierten Zusammensetzung oder eines wie in einem der Ansprüche 1 bis 5 definierten Pyrazolin-4,5-dions aufzutragen.

13. Verwendung eines wie in einem der Ansprüche 1 bis 5 definierten Pyrazolin-4,5-dions in oder für die Herstellung von Zusammensetzungen, die für die Färbung der Haare vorgesehen sind.

14. Verbindungen der folgenden allgemeinen Formel (I') 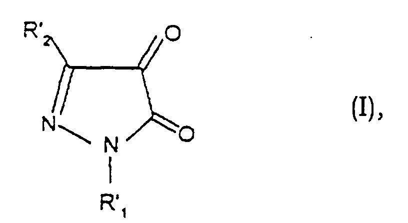 in der R'₁ und R'₂ dieselbe Bedeutung wie R₁ und R₂ in Formel (I) von Anspruch 1 haben, jedoch mit der Maßgabe, daß
(i) R'₂ verschieden von Wasserstoff, Methyl, *tert*.-Butyl, Phenyl, p-Methylphenyl, p-Nitrophenyl und p-Methoxyphenyl ist, wenn R'₁ Phenyl bedeutet,
(ii) R'₁ verschieden von p-Nitrophenyl, 2-Phenoxyethyl, 2-Naphthyloxyethyl, Methyl, m-Trifluormethylbenzyl, 1-Phenylethyl, p-Methylphenyl, 1-Cyclohexylethyl und 1-(3',4'-Dichlorphenyl)-ethyl ist, wenn R'₂ Methyl bedeutet,
(iii) R'₂ verschieden von n-Propyl und Hydroxymethyl ist, wenn R'₁ 2-Naphthyloxyethyl bedeutet,
(iv) R'₂ verschieden von *tert.-*Butyl ist, wenn R'₁ 2-Phenoxyethyl bedeutet,
(v) R'₁ verschieden von 2-Naphthyloxyethyl und 1-(3',4'-Dichlorphenyl)-ethyl ist, wenn R'₂ Methoxymethyl bedeutet,
(vi) R'₂ verschieden von Methyl ist, wenn R'₁ Wasserstoff bedeutet,
(vii) R'₁ verschieden von Methyl, p-Methoxyphenyl und p-Nitrophenyl ist, wenn R'₂ Phenyl bedeutet.

15. Verbindungen nach Anspruch 14, dadurch gekennzeichnet, daß R'₁ ausgewählt ist unter
- Wasserstoff, geradkettigem oder verzweigtem C₁-C₈-Alkyl,
- dem Rest -(CH₂)₂-OR', worin R' Phenyl oder Naphthyl bedeutet,
- dem Rest -(CH₂)_{q}-R", worin q die Zahl 1 oder 2 ist und R" Phenyl bedeutet, das gegebenenfalls mit einer Trifluormethylgruppe substituiert ist,
- Phenyl, das gegebenenfalls mit einer Nitrogruppe, Chlor, C₁-C₄-Alkyl oder C₁-C₄-Alkoxy substituiert ist.

16. Verbindungen nach Anspruch 14 oder 15, dadurch gekennzeichnet, daß R'₂ ausgewählt ist unter
- Wasserstoff, geradkettigem oder verzweigtem C₁-C₄-Alkyl, C₁-C₄-Hydroxyalkyl, Methoxymethyl,
- Phenyl, das gegebenenfalls mit einem Halogenatom, einer Nitrogruppe, C₁-C₄-Alkyl oder C₁-C₄-Alkoxy substituiert ist,
- C₁-C₄-Alkoxy, Trifluormethyl, Acetamido, C₁-C₄-Dialkylamino, Carboxy, Methoxycarbonyl, Ethoxycarbonyl,
- Thienyl, Furyl, Pyridyl und Pyrazolyl.

17. Verbindungen nach Anspruch 16, dadurch gekennzeichnet, daß R₁ unter Wasserstoff, Methyl, Ethyl, Isopropyl, *tert*.-Butyl und Phenyl ausgewählt ist und daß R₂ unter Wasserstoff, Methyl, Phenyl, Methoxyphenyl, Methoxy, Ethoxy, Carboxy, Methoxycarbonyl, Ethoxycarbonyl, Acetamido, Dimethylamino, Diethylamino, Trifluormethyl, Furyl, Pyridyl ausgewählt ist.

18. Verbindung nach Anspruch 17, dadurch gekennzeichnet, daß sie ausgewählt ist unter: 1-Ethyl-3-methylpyrazolin-4,5-dion, 1-Isopropyl-3-methylpyrazolin-4,5-dion, 1-*tert*. -Butyl-3-methylpyrazolin-4,5-dion, 1-Methyl-3-(3'-methoxyphenyl)-pyrazolin-4,5-dion, 3-(2'-Furyl)-1-methylpyrazolin-4,5-dion, 1-Methyl-3-(4'-pyridyl)-pyrazolin-4,5-dion, 1-Methyl-3-methoxypyrazolin-4,5-dion, 3-Ethoxy-1-methylpyrazolin-4,5-dion, 3-Dimethylamino-1-methylpyrazolin-4,5-dion, 3-Diethylamino-1-methylpyrazolin-4,5-dion, 3-Acetamido-1-methylpyrazolin-4,5-dion, 1-Methylpyrazolin-4,5-dion, 1-Ethylpyrazolin-4,5-dion, 1-Isopropylpyrazolin-4,5-dion, 1*-tert.*-Butylpyrazolin-4,5-dion, 3-Methoxy-1-phenylpyrazolin-4,5-dion, 3-Ethoxy-1-phenylpyrazolin-4,5-dion, 3-Acetamido-1-phenylpyrazolin-4,5-dion; 3-Dimethylamino-1-phenylpyrazolin-4,5-dion, 3-Diethylamino-1-phenylpyrazolin-4,5-dion, 1-Phenyl-3-trifluormethylpyrazolin-4,5-dion, 1-Methyl-3-trifluormethylpyrazolin-4,5-dion, 1-Isopropyl-3-trifluormethylpyrazolin-4,5-dion, 1-Ethyl-3-trifluormethylpyrazolin-4,5-dion, 3-Trifluormethylpyrazolin-4,5-dion, 3-Carboxy-1-phenylpyrazolin-4,5-dion, 3-Methoxycarbonyl-1-phenylpyrazolin-4,5-dion, 3-Ethoxycarbonyl-1-phenylpyrazolin-4,5-dion, 3-Methoxypyrazolin-4,5-dion, 3-Ethoxypyrazolin-4,5-dion, 3-Carboxy-1-methylpyrazolin-4,5-dion, 3-Methoxycarbonyl-1-methylpyrazolin-4,5-dion, 3-Ethoxycarbonyl-1-methylpyrazolin-4,5-dion.

19. Verfahren zur Herstellung neuer Verbindungen der Formel (I'), dadurch gekennzeichnet, daß es die folgenden Schritte umfaßt, wobei R'₁ und R'₂ wie in Anspruch 14 definiert sind:
i) Umsetzen eines Pyrazolin5-ons (1) mit einer nitrosoaromatischen Verbindung (2) unter Erhalt des entsprechenden 4-Aryliminopyrazolin-5-ons (3): 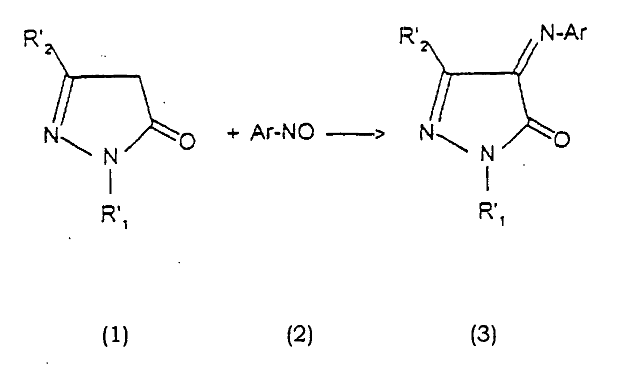 wobei diese Reaktion in einem niederen Alkohol unter Rückfluß bei einer Temperatur von 65 bis 85 °C durchgeführt wird,
(ii) Hydrolysieren des 4-Aryliminopyrazolin-5-ons (3) in stark saurem Medium unter Erhalt des entsprechenden Pyrazolin-4,5-dion-Derivats der Formel (I'):

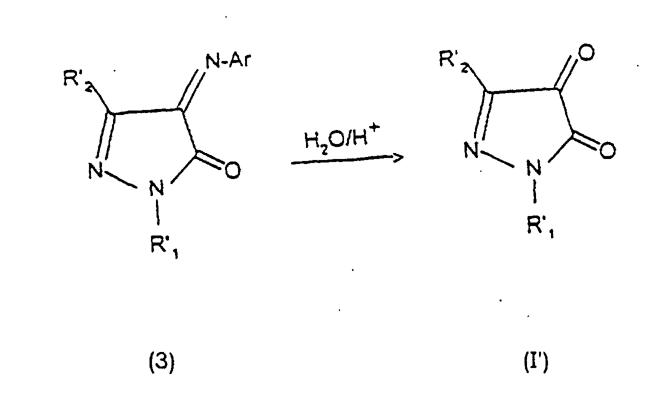

20. Verfahren nach Anspruch 19, dadurch gekennzeichnet, daß der in Schritt (i) verwendete niedere Alkohol unter Methanol, Ethanol und Isopropanol ausgewählt wird.

21. Verfahren nach Anspruch 19 oder 20, dadurch gekennzeichnet, daß der Schritt (i) in Gegenwart einer katalytischen Menge einer schwachen Base, wie eines Carbonats oder Hydrogencarbonats, durchgeführt wird.

22. Verfahren nach einem der Ansprüche 19 bis 21, dadurch gekennzeichnet, daß es sich bei dem nitrosoaromatischen Derivate in Schritt (i) um ein p-Nitrosodialkylanilin der folgenden Formel (2') 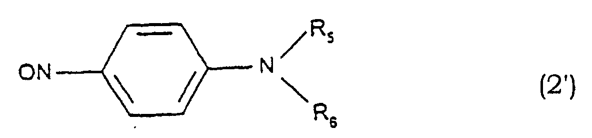 handelt, in der R₅ und R₆ geradkettiges oder verzweigtes C₁-C₄-Alkyl bedeuten.

23. Verfahren nach einem der Ansprüche 19 bis 22, dadurch gekennzeichent, daß die saure Hydrolyse in Schritt (ii) mit verdünnter Schwefelsäure oder wäßriger Chlorwasserstoffsäure bei Umgebungstemperatur in Gegenwart eines nicht mit Wasser mischbaren Colösemittels für das Pyrazolin-4,5-dion durchgeführt wird.

24. Verfahren nach Anspruch 23, dadurch gekennzeichnet, daß es sich bei dem Colösemittel um einen Ether handelt, der unter Diethylether und Diisopropylether ausgewählt wird.

25. Verfahren zur Herstellung neuer Verbindungen der Formel (I'), dadurch gekennzeichnet, daß es die folgenden Schritte umfaßt, wobei R'₁ und R'₂ wie in Anspruch 14 definiert sind:
(i) Umsetzen eines Pyrazolin-5-ons der Formel (1) mit Brom unter Erhalt des entsprechenden 4,4-Dibrompyrazolin-5-ons der Formel (4) (Schritt a),
(ii) Umsetzen des Gemischs mit Bleidiacetat unter Bildung des entsprechenden Diacetats der Formel (5), bei dem es sich um ein instabiles Zwischenprodukt handelt, das spontan unter Eliminierung von Essigsäureanhydrid zum Pyrazolin-4,5-dion der Formel (I') (Schritt b) führt, wobei diese beiden Reaktionsschritte nach dem folgenden Reaktionsschema durchgeführt werden:

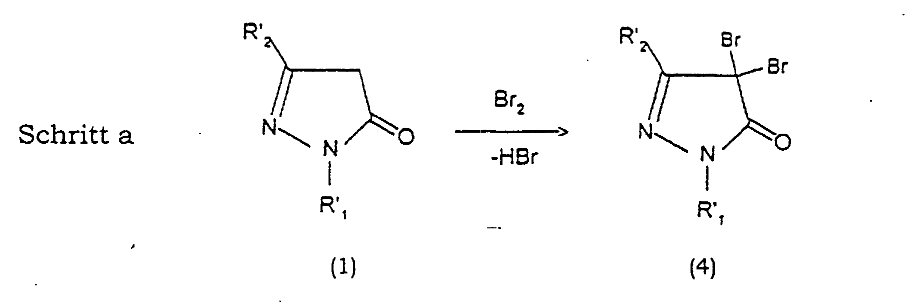

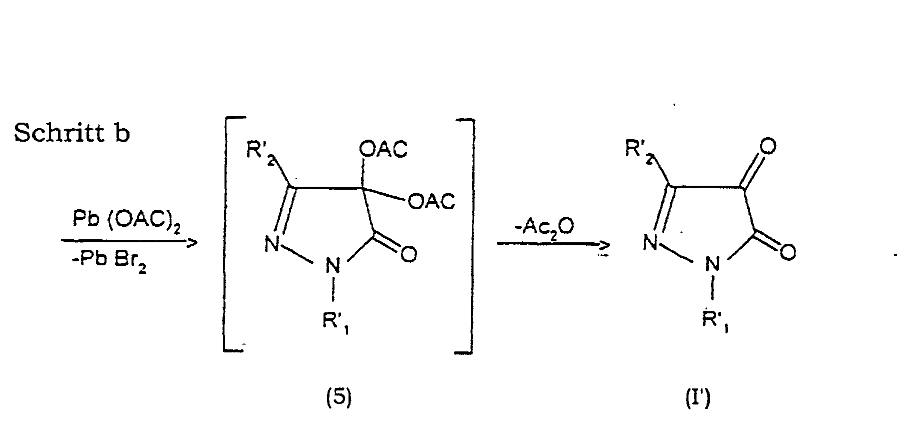

26. Verfahren nach Anspruch 25, dadurch gekennzeichnet, daß der Schritt a in wäßrigem Medium in Gegenwart von zwei Äquivalenten Brom bei Umgebungstemperatur durchgeführt wird.

27. Verfahren nach Anspruch 25 oder 26, dadurch gekennzeichnet, daß der Schritt b während einiger Stunden unter Rückfluß der Essigsäure durchgeführt wird.

## Claims

1. Composition comprising, in a cosmetically acceptable support, at least one 4,5-pyrazolinedione corresponding to formula (I) below: 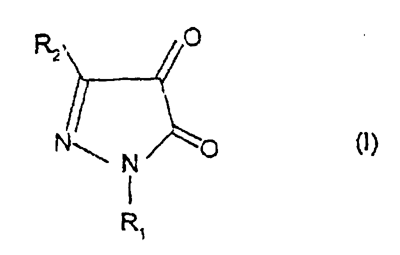 in which R₁ is chosen from:
- a hydrogen atom; a linear or branched C₁-C₁₈ alkyl radical optionally substituted with a hydroxyl (OH), sulphonyl (SO₃H) or carboxyl (COOH) radical; a cyclohexyl or cyclopentyl radical;
- a radical 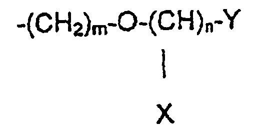 in which m is equal to 1, 2 or 3, n is equal to 1, 2 or 3, X is a hydrogen atom or a methyl radical, and Y is a methyl, hydroxyl or linear or branched C₁-C₅ alkoxy radical;
- a radical -(CH₂)ₚ-OR' in which R' represents a phenyl or naphthyl radical and p is equal to 1 or 2;
- a radical -(CH₂)_{q}-R" in which q is equal to 1, 2 or 3 and R'' is chosen from:
- a phenyl radical which is unsubstituted or substituted with not more than 2 radicals chosen from methyl, trifluoromethyl, methoxy or sulphonyl radicals;
- a naphthyl radical, a thienyl radical, a furyl radical, a pyridyl radical or a piperidyl radical;
- a radical: 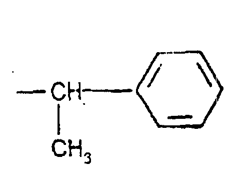
- a radical: 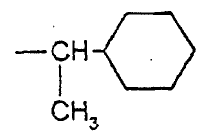
- a radical: 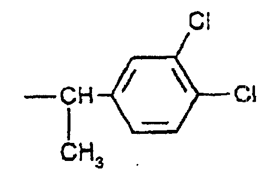
- a phenyl radical; a phenyl radical substituted with a nitro radical; a phenyl radical substituted with one to three radicals chosen from: -COOH, -CH₂COOH, -Cl, -Br, -F, -SO₃H, -CH₂OH, -OCF₃, -CF₃, -SO₂CH₃, -SO₂NH₂, -SO₂NHC₂H₅, -SO₂NHCH₂CH₂OH, -CON(CH₃)₂, -CON(C₂H₅)₂, -CH₂N(CH₃)₂, -CH₂N(C₂H₅)₂, -NHCOCH₃, -NHCOC₂H₅, a C₁-C₈ alkyl radical or a radical -ZR₃ in which Z is an oxygen or sulphur atom and R₃ is a hydrogen atom or a linear or branched C₁-C₁₈ alkyl radical;
- a naphthyl radical optionally substituted with an -SO₃H radical;
- a benzyl radical substituted with a -COOH radical;
- a pyridyl radical, a pyrimidinyl radical, a pyrazinyl radical, a triazinyl radical, a benzotriazolyl radical, a benzimidazolyl radical, a thienyl radical, an imidazolyl radical, a thiazolyl radical, a 1,2,4-triazolyl radical, an indazolyl radical, an indolyl radical, a quinolyl radical or an isoquinolyl radical; and R₂ is chosen from:
- a hydrogen atom; a linear or branched C₁-C₆ alkyl radical; a C₁-C₄ hydroxyalkyl radical;
- a phenyl radical; a phenyl radical substituted with a halogen atom, a nitro radical or a trifluoromethyl radical; a phenyl radical substituted with not more than three radicals chosen from C₁-C₄ alkyl, C₁-C₄ alkoxy, C₁-C₄ dialkylamino and C₁-C₂ alkylthio radicals;
- a benzyl radical; a benzyl radical substituted with a halogen atom, a C₁-C₄ alkyl radical, a C₁-C₄ alkoxy radical, a trifluoromethyl radical or a C₁-C₄ dialkylamino radical;
- a radical -(CH₂)ᵣ-R₄ in which r is equal to 1, 2 or 3 and R₄ is chosen from:
- an -SO₃H radical, a C₁-C₂ alkylthio radical or a benzylthio radical;
- a methoxycarbonyl or ethoxycarbonyl radical;
- a C₁-C₄ alkoxy radical or a phenoxy radical optionally substituted with one or more halogen atoms;
- a C₁-C₄ alkoxy radical; a trifluoromethyl radical; an acetamido radical; a C₁-C₄ dialkylamino radical; a carboxyl radical; a methoxycarbonyl radical; an ethoxycarbonyl radical;
- a thienyl radical, a furyl radical, a pyridyl radical or a pyrazolyl radical.

2. Composition according to Claim 1, characterized in that R₁ is chosen from:
- a hydrogen atom; a linear or branched C₁-C₈ alkyl radical;
- a radical -(CH₂)₂-OR' in which R' represents a phenyl or naphthyl radical;
- a radical -(CH₂)_{q}-R'' in which q is equal to 1 or 2 and R'' represents a phenyl radical optionally substituted with a trifluoromethyl radical;
- a phenyl radical optionally substituted with a nitro radical, a -CI radical, a C₁-C₄ alkyl radical or a C₁-C₄ alkoxy radical.

3. Composition according to Claim 1 or 2, characterized in that R₂ is chosen from:
- a hydrogen atom; a linear or branched C₁-C₄ alkyl radical; a C₁-C₄ hydroxyalkyl radical; a methoxymethyl radical;
- a phenyl radical optionally substituted with a halogen atom, a nitro radical, a C₁-C₄ alkyl radical or a C₁-C₄ alkoxy radical;
- a C₁-C₄ alkoxy radical; a trifluoromethyl radical; an acetamido radical; a C₁-C₄ dialkylamino radical; a carboxyl radical; a methoxycarbonyl radical; an ethoxycarbonyl radical;
- a thienyl radical, a furyl radical, a pyridyl radical or a pyrazolyl radical.

4. Composition according to Claim 3, characterized in that R₁ is chosen from:
- hydrogen and methyl, ethyl, isopropyl, tert-butyl and phenyl radicals;
and R₂ is chosen from:
- hydrogen and methyl, phenyl, methoxyphenyl, methoxy, ethoxy, carboxyl, methoxycarbonyl, ethoxycarbonyl, acetamido, dimethylamino, diethylamino, trifluoromethyl, furyl and pyridyl radicals.

5. Composition according to Claim 4, characterized in that the 4,5-pyrazolinedione is chosen from: 3-methyl-1-phenyl-4,5-pyrazolinedione; 3-methyl-4,5-pyrazolinedione; 1,3-dimethyl-4,5-pyrazolinedione; 1-ethyl-3-methyl-4,5-pyrazolinedione; 1-isopropyl-3-methyl-4,5-pyrazolinedione; 1-tert-butyl-3-methyl-4,5-pyrazolinedione; 1-methyl-3-phenyl-4,5-pyrazolinedione; 1-methyl-3-(3'-methoxyphenyl)-4,5-pyrazolinedione; 3-(2'-furyl)-1-methyl-4,5-pyrazolinedione; 1-methyl-3-(4'-pyridyl)-4,5-pyrazolinedione; 1-methyl-3-methoxy-4,5-pyrazolinedione; 3-ethoxy-1-methyl-4,5-pyrazolinedione; 3-dimethylamino-1-methyl-4,5-pyrazolinedione; 3-diethylamino-1-methyl-4,5-pyrazolinedione; 3-acetamido-1-methyl-4,5-pyrazolinedione; 1-phenyl-4,5-pyrazolinedione; 1-methyl-4,5-pyrazolinedione; 1-ethyl-4,5-pyrazolinedione; 1-isopropyl-4,5-pyrazolinedione; 1-tert-butyl-4,5-pyrazolinedione; 3-methoxy-1-phenyl-4,5-pyrazolinedione; 3-ethoxy-1-phenyl-4,5-pyrazolinedione; 3-acetamido-1-phenyl-4,5-pyrazolinedione; 3-dimethylamino-1-phenyl-4,5-pyrazolinedione; 3-diethylamino-1-phenyl-4,5-pyrazolinedione; 1-phenyl-3-trifluoromethyl-4,5-pyrazolinedione; 1-methyl-3-trifluoromethyl-4,5-pyrazolinedione; 1-isopropyl-3-trifluoromethyl-4,5-pyrazolinedione; 1-ethyl-3-trifluoromethyl-4,5-pyrazolinedione; 3-trifluoromethyl-4,5-pyrazolinedione; 3-carboxy-1-phenyl-4,5-pyrazolinedione; 3-methoxycarbonyl-1-phenyl-4,5-pyrazolinedione; 3-ethoxycarbonyl-1-phenyl-4,5-pyrazolinedione; 3-methoxy-4,5-pyrazolinedione; - 3-ethoxy-4,5-pyrazolinedione; 3-carboxy-1-methyl-4,5-pyrazolinedione; 3-methoxycarbonyl-1-methyl-4,5-pyrazolinedione; 3-ethoxycarbonyl-1-methyl-4,5-pyrazolinedione.

6. Composition according to any one of Claims 1 to 5, characterized in that the 4,5-pyrazolinedione is present in the composition at a concentration of between 0.05 and 10% by weight relative to the total weight of the composition.

7. Composition according to Claim 6, characterized in that the said concentration is between 0.05 and 5% by weight relative to the total weight of the composition.

8. Composition according to any one of the preceding claims, characterized in that it is a cosmetic composition for colouring the skin.

9. Composition according to any one of Claims 1 to 7, characterized in that it is a cosmetic composition for dyeing the hair.

10. Cosmetic treatment process for the skin which is intended to artificially tan and/or brown it,
characterized in that it consists in applying an effective amount of a composition as defined in any one of Claims 1 to 8 or a 4,5-pyrazolinedione as defined in any one of Claims 1 to 5 to the skin.

11. Use of a 4,5-pyrazolinedione as defined in any one of Claims 1 to 5 in, or for the manufacture of, compositions intended to artificially tan and/or brown the skin.

12. Cosmetic treatment process for the hair which is intended to dye it, characterized in that it consists in applying an effective amount of a composition as defined in any one of Claims 1 to 9 or a 4,5-pyrazolinedione as defined in any one of Claims 1 to 5 to the hair.

13. Use of a 4,5-pyrazolinedione as defined in any one of Claims 1 to 5 in, or for the manufacture of, compositions intended to dye the hair.

14. Compounds corresponding to the general formula (I') below: 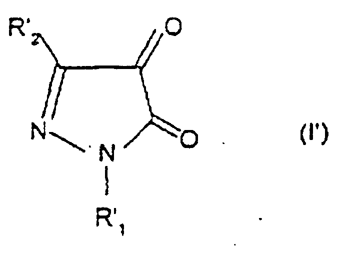 in which R'₁ and R'₂ have the same meanings as those given in formula (I) of Claim 1 for R₁ and R₂ with, however, the following provisos:
- (i) when R'₁ represents a phenyl radical, then R'₂ is other than a hydrogen atom, a methyl radical, a tert-butyl radical, a phenyl radical, a p-methylphenyl radical, a p-nitrophenyl radical or a p-methoxyphenyl radical,
- (ii) when R'₂ represents a methyl radical, R'₁ is other than a p-nitrophenyl radical, a 2-phenoxyethyl radical, a 2-naphthyloxyethyl radical, a methyl radical, an m-trifluoromethylbenzyl radical, a 1-phenylethyl radical, a p-methylphenyl radical, a 1-cyclohexylethyl radical or a 1-(3',4'-dichlorophenyl)ethyl radical,
- (iii) when R'₁ represents a 2-naphthyloxyethyl radical, R'₂ is other than an n-propyl radical or a hydroxymethyl radical,
- (iv) when R'₁ represents a 2-phenoxyethyl radical, R'₂ is other than a tert-butyl radical,
- (v) when R'₂ represents a methoxymethyl radical, R'₁ is other than a 2-naphthyloxyethyl radical or a 1- (3',4'-dichlorophenyl)ethyl radical,
- (vi) when R'₁ represents a hydrogen atom, R'₂ is other than a methyl radical.
- (vii) when R'₂ represents a phenyl radical, R'₁ is other than a methyl radical, a p-methoxyphenyl radical or a p-nitrophenyl radical.

15. Compounds according to Claim 14, characterized in that R'₁ is chosen from:
- a hydrogen atom; a linear or branched C₁-C₈ alkyl radical;
- a radical -(CH₂)₂-OR' in which R' represents a phenyl or naphthyl radical;
- a radical - (CH₂)_{q}-R' ' in which q is equal to 1 or 2 and R'' represents a phenyl radical optionally substituted with a trifluoromethyl radical;
- a phenyl radical optionally substituted with a nitro radical, a -Cl radical, a C₁-C₄ alkyl radical or a C₁-C₄ alkoxy radical.

16. Compounds according to Claim 14 or 15, characterized in that R'₂ is chosen from:
- a hydrogen atom; a linear or branched C₁-C₄ alkyl radical; a C₁-C₄ hydroxyalkyl radical; a methoxymethyl radical;
- a phenyl radical optionally substituted with a halogen atom, a nitro radical, a C₁-C₄ alkyl radical or a C₁-C₄ alkoxy radical;
- a C₁-C₄ alkoxy radical; a trifluoromethyl radical; an acetamido radical; a C₁-C₄ dialkylamino radical; a carboxyl radical; a methoxycarbonyl radical; an ethoxycarbonyl radical;
- a thienyl radical, a furyl radical, a pyridyl radical or a pyrazolyl radical.

17. Compounds according to Claim 16, characterized in that R'₁ is chosen from:
- hydrogen and methyl, ethyl, isopropyl, tert-butyl and phenyl radicals;
and R'₂ is chosen from:
- hydrogen and methyl, phenyl, methoxyphenyl, methoxy, ethoxy, carboxyl, methoxycarbonyl, ethoxycarbonyl, acetamido, dimethylamino, diethylamino, trifluoromethyl, furyl and pyridyl radicals.

18. Compound according to Claim 17, characterized in that it is chosen from: 1-ethyl-3-methyl-4,5-pyrazolinedione; 1-isopropyl-3-methyl-4,5-pyrazolinedione; 1-tert-butyl-3-methyl-4,5-pyrazolinedione; 1-methyl-3-(3'-methoxyphenyl)-4,5-pyrazolinedione; 3- (2' -furyl)-1-methyl-4,5-pyrazolinedione; 1-methyl-3-(4'-pyridyl)-4,5-pyrazolinedione; 1-methyl-3-methoxy-4,5-pyrazolinedione; 3-ethoxy-1-methyl-4,5-pyrazolinedione; 3-dimethylamino-1-methyl-4,5-pyrazolinedione; 3-diethylamino-1-methyl-4,5-pyrazoli.nedione; 3-acetamido-1-methyl-4,5-pyrazolinedione; 1-methyl-4,5-pyrazolinedione; 1-ethyl-4,5-pyrazolinedione; 1-isopropyl-4,5-pyrazolinedione; 1-tert-butyl-4,5-pyrazolinedione; 3-methoxy-1-phenyl-4,5-pyrazolinedione; 3-ethoxy-1-phenyl-4,5-pyrazolinedione; 3-acetamido-1-phenyl-4,5-pyrazolinedione; 3-dimethylamino-1-phenyl-4,5-pyrazolinedione; 3-diethylamino-1-phenyl-4,5-pyrazolinedione; 1-phenyl-3-trifluoromethyl-4,5-pyrazolinedione; 1-methyl-3-trifluoromethyl-4,5-pyrazolinedione; 1-isopropyl-3-trifluoromethyl-4,5-pyrazolinedione; 1-ethyl-3-trifluoromethyl-4,5-pyrazolinedione; 3-trifluoromethyl-4,5-pyrazolinedione; 3-carboxy-1-phenyl-4,5-pyrazolinedione; 3-methoxycarbonyl-1-phenyl-4,5-pyrazolinedione; 3-ethoxycarbonyl-1-phenyl-4,5-pyrazolinedione; 3-methoxy-4,5-pyrazolinedione; 3-ethoxy-4,5-pyrazolinedione; 3-carboxy-1-methyl-4,5-pyrazolinedione; 3-methoxycarbonyl-1-methyl-4,5-pyrazolinedione; 3-ethoxycarbonyl-1-methyl-4,5-pyrazolinedione.

19. Process for the preparation of the novel compounds of formula (I'), characterized in that it comprises the following steps, the meanings of R'₁ and R'₂ being as defined in Claim 14:
i) a 5-pyrazolinone (1) is reacted with an aromatic nitroso compound (2) in order to obtain the corresponding 4-arylimino-5-pyrazolinone (3) : 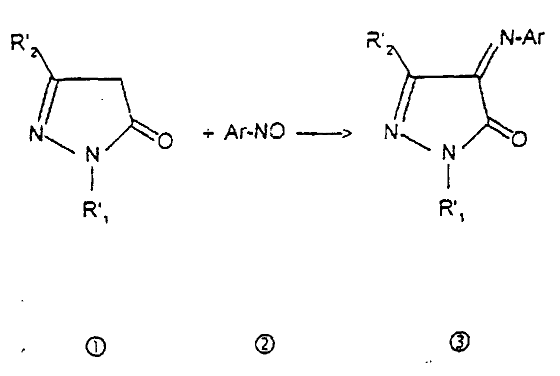 this reaction being carried out in a lower alcohol, at reflux, at a temperature of between 65°C and 85°C,
ii) the 4-arylimino-5-pyrazolinone (3) is then hydrolysed, in strong acid medium, in order to obtain the corresponding 4,5-pyrazolinedione derivative of formula (I') :

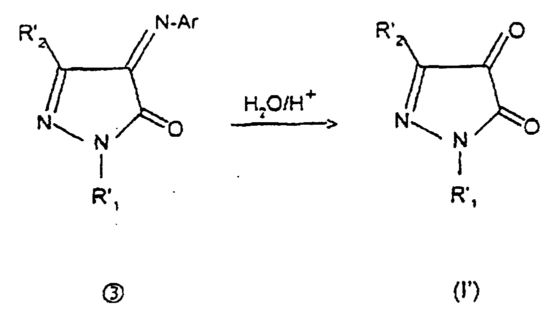

20. Process according to Claim 19, characterized in that the lower alcohol used in step (i) is chosen from methanol, ethanol and isopropanol.

21. Process according to Claim 19 or 20, characterized in that step (i) is carried out in the presence of a catalytic amount of a weak base such as a carbonate or a bicarbonate.

22. Process according to any one of Claims 19 to 21, characterized in that the aromatic nitroso derivative of step (i) is a p-nitrosodialkylaniline of formula (2)' below: 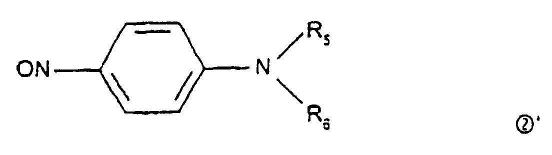 in which R₅ and R₆ represent a linear or branched C₁-C₄ alkyl radical.

23. Process according to any one of Claims 19 to 22, characterized in that the acid hydrolysis of step (ii) is carried out with dilute sulphuric acid or aqueous hydrochloric acid at room temperature, in the presence of a co-solvent for the 4,5-pyrazolinedione, which is immiscible with water.

24. Process according to Claim 23, characterized in that the said co-solvent is an ether chosen from diethyl ether and diisopropyl ether.

25. Process for the preparation of the novel compounds of formula (I'), characterized in that it comprises the following steps, the meanings of R'₁ and R'₂ being as defined in Claim 14:
i) bromine is reacted with a 5-pyrazolinone of formula (1) in order to obtain the corresponding 4,4-dibromo-5-pyrazolinone of formula (4) (step a),
ii) lead diacetate is then reacted so as to form the corresponding diacetate of formula (5), this product being an unstable intermediate which leads spontaneously, by elimination of acetic anhydride, to the 4,5-pyrazolinedione derivative of formula (I') (step b), these two steps being carried out according to the following reaction scheme:

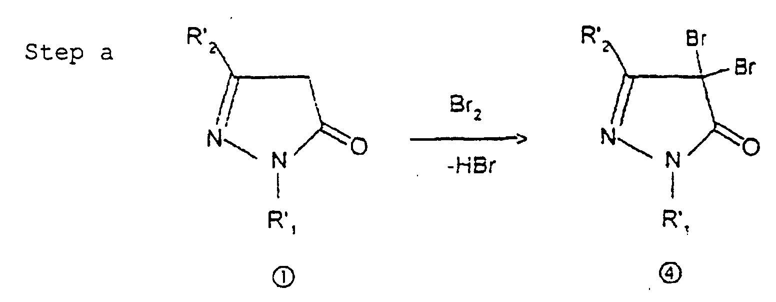

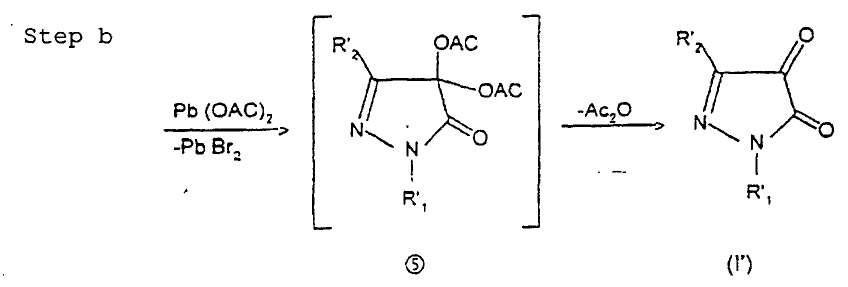

26. Process according to Claim 25, characterized in that step a is carried out in aqueous medium in the presence of two equivalents of bromine at room temperature.

27. Process according to Claim 25 or 26, characterized in that step b is carried out at the reflux point of acetic acid for a few hours.
